# EUROPÄISCHE PATENTSCHRIFT

(11) **EP 3 750 509 B1**
(45) Veröffentlichungstag und Bekanntmachung des Hinweises auf die Patenterteilung: **14.02.2024**
(21) Anmeldenummer: 20176842.1
(22) Anmeldetag: 27.05.2020
(51) Int. Cl.: A61F 2/28, A61M 27/00, A61B 17/70

(54) **MEDIZINISCH EINSETZBARER PLATZHALTER**
MEDICALLY INSERTABLE SPACER
DISPOSITIF DE MAINTIEN D'ESPACE À USAGE MÉDICAL

(30) Priorität: 12.06.2019 DE 102019115932
(43) Veröffentlichungstag der Anmeldung: 16.12.2020
(73) Patentinhaber: Heraeus Medical GmbH, 61273 Wehrheim (DE)
(72) Erfinder: Vogt, Sebastian, 61273 Wehrheim (DE); Kluge, Thomas, 61273 Wehrheim (DE); Büchner, Hubert, 61273 Wehrheim (DE)
(74) Vertreter: Schultheiss & Sterzel Patentanwälte PartG mbB

(56) Entgegenhaltungen:
- WO-A2-2007/106591
- US-A- 5 219 326
- US-A1- 2001 004 710
- KATSUJI MATSUNAGA ET AL: "Gas Permeability of Thermoplastic Polyurethane Elastomers", POLYMER JOURNAL, Bd. 37, Nr. 6, 15. Juni 2005 (2005-06-15), Seiten 413-417, XP055379460, JP ISSN: 0032-3896, DOI: 10.1295/polymj.37.413

## Beschreibung

Die Erfindung betrifft einen medizinischen Platzhalter zur Behandlung von Knochendefekten. Die Erfindung betrifft auch ein Verfahren zum Begasen einer Oberfläche eines medizinischen Platzhalters.

Gegenstand der Erfindung ist erfindungsgemäß ein expandierbarer medizinischer Platzhalter zur temporären Implantation in Knochenkavitäten. Der medizinische Platzhalter ist neben seiner Platzhaltungsfunktion auch zum Gasaustausch mit dem umliegenden Gewebe bestimmt, wobei Sauerstoff oder ein Sauerstoff enthaltendes Spülgasgemisch oder eine mit Sauerstoff angereicherte Spülflüssigkeit den Innenraum des Abstandhalters kontinuierlich oder diskontinuierlich durchströmt und über die permeable Außenwand des Platzhalters dem Gewebe Sauerstoff zugeführt und gleichzeitig Kohlendioxid abgeführt werden kann.

Aus der US 4 576 590 ist ein Katheter mit einer für Sauerstoff durchlässigen Wandung einer Röhre bekannt, der zur Behandlung von inneren Organen vorgesehen ist. Dabei darf das Material der verwendeten Röhre sich möglichst nicht ausdehnen, da dies die inneren Organe beeinträchtigen könnte. Die Vorrichtung ist dementsprechend nicht als variabler Platzhalter für Knochendefekte einsetzbar. Die US 2016/0235902 A1 offenbart ein Gasaustauschsystem für ein Körperlumen (die Luftröhre), bei dem ein Gasaustausch zwischen der Luftröhre und einer Membran des Gasaustauschsystems erfolgen kann. Auch dieses System ist nicht zur Behandlung von Knochendefekten verwendbar.

Die US 2001/0004710 A1 offenbart einen Platzhalter zum Behandeln einer Kavität eines Patienten, bei dem ein Ballon aufgeblasen werden kann und in den Ballon ein härtbares Biomaterial eingespeist werden kann. Ein Gasaustausch ist nicht vorgesehen. In dem Artikel "Gas Permeability of Thermoplastic Polyurethane Elastomers" von Katuji Matsunaga et a. in Polymer Jounal Vol. 37, No. 6, Seiten 413-417 (2205) ist ein Kunststoff bekannt, der für Sauerstoff und CO₂ durchlässig ist.

Größere Segmentdefekte von Röhrenknochen stellen auch heute noch eine große Herausforderung für Chirurgen dar. Segmentdefekte können unter anderem durch Traumata, infizierte Pseudoarthrosen und durch chronische Osteitis verursacht werden. Diese Knochendefekte werden vielfach mit Hilfe der Illizarov-Methode therapiert. Für die Behandlung großer dia- und metaphysärer Knochendefekte, mit einer Defektgröße > 3 cm, stellt auch die Masquelet-Technik eine Behandlungsoption dar. Dabei wird in einer ersten Operation der Knochendefekt ausgiebig debridiert und ein Knochenzementspacer als Platzhalter in den Markraum eingesetzt. Der Knochenzementspacer wird mit Weichgewebe abgedeckt. Eine ausreichende Weichteilteildeckung ist für den Behandlungserfolg unbedingt notwendig. Die mechanische Stabilisierung des Knochendefekts erfolgt durch einen Fixateur Externe. Im Zeitraum von vier bis sechs Wochen bildet sich um den Knochenzementspacer eine vaskularisierte Bindegewebsmembran aus. Es wird damit das Periost nachgebildet. Danach wird der Knochenzementspacer in einer zweiten Operation entfernt. Dazu erfolgt üblicherweise mindestens eine Längsinzision. Es wird dabei versucht, die gebildete Bindegewebsmembran soweit als möglich zu schonen, was jedoch schwierig ist, da der Knochenzementspacer einen Durchmesser gleich dem Durchmesser der Markhöhle hat. Danach wird der Markraum mit autologer Spongiosa, Knochenersatzmaterialien oder Gemischen aus autologer Spongiosa und Knochenersatzmaterialien aufgefüllt. Es erfolgt dann in den meisten Fällen eine membrangeleitete Ossifikation entlang der Bindegewebsmembran.

Nachteilig ist also, dass beim Entfernen des Knochenzementspacers beziehungsweise des Platzhalters die Bindegewebsmembran beeinträchtigt wird, die nachfolgend für die Ossifikation benötigt wird. Zudem wäre es wünschenswert, wenn die Bindegewebsmembran sich schnell und stabil aufbaut, um den nachfolgenden Heilungsprozess zu unterstützen. Gleichzeitig sollte der Platzhalter variabel an unterschiedliche Behandlungssituationen anpassbar sein.

Die Erfindung hat die Aufgabe einen medizinischen Abstandhalter zu entwickeln, der als temporäres Implantat in Knochenkavitäten oder Knochendefekten implantiert werden kann. Das Volumen und die Gestalt des Abstandhalters sollen möglichst variiert werden können. Der Abstandhalter soll so aus den Knochenkavitäten oder Knochendefekten entfernbar sein, dass nur eine möglichst geringe Schädigung des anliegenden Gewebes auftritt. Die den Abstandhalter umgebende Bindegewebsmembran soll möglichst unbeschädigt erhalten bleiben. Der Abstandhalter soll weiterhin einen Gasaustausch mit dem humanen Gewebe ermöglichen, das an dem Abstandhalter unmittelbar nach der Implantation anliegt oder sich dort während des Implantationszeitraums bildet. Das Gewebe soll mit Sauerstoff versorgt werden können und gleichzeitig soll das beim Stoffwechsel des Gewebes anfallende Kohlendioxid kontinuierlich oder auch diskontinuierlich entfernt werden können. Der Abstandhalter soll als temporäres Implantat für die Masquele-Technik im Rahmen der Behandlung von großen Knochendefekten geeignet sein.

Die Aufgaben der Erfindung werden gelöst durch einen medizinischen Platzhalter aufweisend mindestens einen Hohlkörper, der expandierbar ist und der einen Innenraum im Inneren des Hohlkörpers begrenzt, einen Gaseinleitungsschlauch, der mit dem Innenraum des Hohlkörpers gasdurchlässig verbunden oder verbindbar ist, einen Gasausleitungsschlauch, der mit dem Innenraum des Hohlkörpers gasdurchlässig verbunden oder verbindbar ist, wobei der Hohlkörper zumindest bereichsweise oder vollständig aus zumindest einem Kunststoff besteht, wobei der Hohlkörper oder der zumindest eine Kunststoff des Hohlkörpers einen Permeabilitätskoeffizienten für Sauerstoff von größer oder gleich 0,5 cm³/(m²*d*bar) und einen Permeabilitätskoeffizienten für Kohlendioxid von größer oder gleich 0,5 cm³/(m²*d*bar) aufweist und wobei in dem Gaseinleitungsschlauch ein Einwegventil angeordnet ist und in dem Gasausleitungsschlauch ein Überdruckventil angeordnet ist.

Bevorzugt kann vorgesehen sein, dass der medizinische Platzhalten zur temporären Implantation in Knochenkavitäten geeignet ist.

Bevorzugt kann auch vorgesehen sein, dass der Hohlkörper aus einem für Sauerstoff und Kohlendioxid permeablen Kunststoff gefertigt ist.

Des Weiteren kann vorgesehen sein, dass der Hohlkörper zumindest so stark expandierbar ist, dass sich das Volumen des Hohlkörpers um zumindest 50% vergrößern lässt, bevorzugt um zumindest 100% vergrößern lässt, besonders bevorzugt um zumindest 200% vergrößern lässt.

Ferner kann vorgesehen sein, dass der Hohlkörper zumindest so stark elastisch expandierbar ist, dass sich das Volumen des Hohlkörpers reversibel und zerstörungsfrei um zumindest 50% vergrößern lässt, bevorzugt um zumindest 100% vergrößern lässt, besonders bevorzugt um zumindest 200% vergrößern lässt.

Bevorzugt kann ferner vorgesehen sein, dass der Innenraum zur Umgebung des medizinischen Platzhalters abgeschlossen ist.

Gemäß einer bevorzugten Weiterbildung kann vorgesehen sein, dass der zumindest eine Kunststoff für Flüssigkeiten undurchlässig ist.

Der Hohlkörper beziehungsweise der Kunststoffhohlkörper bildet mit dem Gaseinleitungsschlauch und dem Gasausleitungsschlauch einen vorzugsweise zur Umgebung abgeschlossenen gemeinsamen Innenraum, so dass bei Einleitung von unter Druck stehendem Spülgas (beziehungsweise einem Spülgasgemisch) oder von einer Sauerstoff enthaltenden Spülflüssigkeit der Hohlkörper expandieren kann. Dazu ist es vorteilhaft, wenn die Wandung des Hohlkörpers aus einem gummielastischen Kunststoff oder einem plastisch verformbaren Kunststoff gebildet ist, so dass der Hohlkörper aufgeblasen beziehungsweise expandiert werden kann. Damit ist es möglich, die Gestalt des Hohlkörpers und damit des medizinischen Platzhalters an die Gestalt des temporär zu füllenden Knochendefekts beziehungsweise des temporär zu füllenden Hohlraums anzupassen.

Der zumindest eine Kunststoff bildet bevorzugt zumindest bereichsweise eine durchgehende Wandung des Hohlkörpers. Das heißt, dass es Bereiche der Wandung des Hohlkörpers gibt, in denen außer dem zumindest einen Kunststoff aus keinem anderen zusätzlichen Material besteht. Gut durchlässige Netze und Drähte, insbesondere aus Metall, stören dabei nicht. Hierdurch soll sichergestellt werden, dass die Durchlässigkeit des Kunststoffs für Sauerstoff und Kohlendioxid dazu genutzt werden kann, dass die Wandung des Hohlkörpers zumindest in diesen Bereichen ebenfalls für Sauerstoff und Kohlendioxid durchlässig ist.

Bevorzugt kann also vorgesehen sein, dass eine den Innenraum des Hohlkörpers begrenzende Wandung für Sauerstoff und Kohlendioxid durchlässig ist.

Gemäß einer bevorzugten Ausführung der vorliegenden Erfindung kann vorgesehen sein, dass der Hohlkörper röhrenförmig ausgebildet ist, wobei der Gaseinleitungsschlauch und der Gasausleitungsschlauch bevorzugt an einer Stirnseite des Hohlkörpers mit dem Innenraum des Hohlkörpers verbunden sind oder an zwei einander gegenüberliegenden Stirnseiten des Hohlkörpers mit dem Innenraum des Hohlkörpers verbunden sind. Röhrenförmige Hohlkörper sind als Abstandshalter insbesondere bei Knochendefekten der langen Röhrenknochen besonders gut geeignet.

Der Hohlkörper kann auch jede andere beliebige Gestalt aufweisen, je nach der Größe und Gestalt des temporär auszufüllenden Knochendefekts.

Bei der vorliegenden Erfindung kann auch vorgesehen sein, dass der Hohlkörper oder der zumindest eine Kunststoff des Hohlkörpers einen Permeabilitätskoeffizienten für Sauerstoff von größer oder gleich 1 cm³/(m²*d*bar) und einen Permeabilitätskoeffizienten für Kohlendioxid von größer oder gleich 1 cm³/(m²*d*bar) aufweist.

Hierdurch kann eine gute Versorgung der Bindegewebsmembran mit Sauerstoff und ein guter Abtransport von Kohlendioxid von der Bindegewebsmembran erfolgen und dabei von der Innenseite der Bindegewebsmembran erfolgen.

Der Permeabilitätskoeffizient wird nach DIN 53380-4 (11/2006) bestimmt. Diese betrifft insbesondere die Prüfung von Kunststoffen, hier die Bestimmung der Gasdurchlässigkeit, wobei im Teil 4 der DIN 53380 ein Kohlenstoffdioxid-spezifisches Infrarotabsorptions-Verfahren zur Messung an Kunststoff-Folien und Kunststoff-Formteilen standardisiert wird, das auch auf Sauerstoff übertragbar ist. Derartige Messungen werden beispielsweise von der Firma Mecadi GmbH (Bexbach, Deutschland) durchgeführt und angeboten.

Es kann also bevorzugt vorgesehen sein, dass der Hohlkörper oder der zumindest eine Kunststoff des Hohlkörpers einen Permeabilitätskoeffizienten für Sauerstoff von größer oder gleich 0,5 cm³/(m²*d*bar) und einen Permeabilitätskoeffizienten für Kohlendioxid von gleich/größer 0,5 cm³/(m²*d*bar) gemäß DIN 53380-4 (11/2006) aufweist, bevorzugt einen Permeabilitätskoeffizienten für Sauerstoff von größer oder gleich 1 cm³/(m²*d*bar) und einen Permeabilitätskoeffizienten für Kohlendioxid von größer oder gleich 1 cm³/(m²*d*bar) gemäß DIN 53380-4 (11/2006) aufweist.

Permeabilität bezeichnet allgemein bei Festkörpern die Eigenschaft, Gase und/oder Flüssigkeiten passieren zu lassen. Vorliegend bezieht sich dies auf die Permeabilität der Wandungen des Hohlkörpers und dies bezogen auf molekularen Sauerstoff und molekulares Kohlendioxid in gasförmiger Form. Der Permeabilitätskoeffizient ist eine materialspezifische Konstante und ist ein Maß der Durchlässigkeit für Flüssigkeiten und Gase.

Die Einheit d steht für einen Tag (day).

Des Weiteren kann vorgesehen sein, dass eine Einströmöffnung des Gaseinleitungsschlauchs, mit der der Gaseinleitungsschlauch in den Innenraum mündet, räumlich getrennt von einer Ausströmöffnung des Gasausleitungsschlauchs angeordnet ist, wobei die Ausströmöffnung die Einmündung des Innenraums in den Gasausleitungsschlauch bildet.

Hierdurch wird beim Durchströmen des Hohlkörpers ein ausreichend langer Kontakt des durchströmenden Spülgases oder der durchströmenden Spülflüssigkeit mit der Innenwand des Hohlkörpers oder des zumindest einen Kunststoffs erreicht, wodurch ein Gasaustausch von Sauerstoff und Kohlendioxid durch die Wand des Hohlkörpers oder des zumindest einen Kunststoffs begünstigt wird.

Die Einströmöffnung ist die Öffnung des Gaseinleitungsschlauchs, durch die das Spülgas oder die Spülflüssigkeit aus dem Gaseinleitungsschlauch in den Innenraum des Hohlkörpers einströmt. Dementsprechend ist die Ausströmöffnung die Öffnung des Gasausleitungsschlauchs, durch die das Spülgas oder die Spülflüssigkeit in den Gasausleitungsschlauch aus dem Innenraum des Hohlkörpers ausströmt.

Dabei kann vorgesehen sein, dass die Einströmöffnung des Gaseinleitungsschlauchs an einem ersten Ende des Hohlkörpers angeordnet ist und die Ausströmöffnung an einem dem ersten Ende gegenüberliegenden zweiten Ende des Hohlkörpers angeordnet ist.

Dadurch wird die Verweilzeit des durchströmenden Spülgases oder der durchströmenden Spülflüssigkeit an der Innenwand des Hohlkörpers beziehungsweise an der Innenwand des zumindest einen Kunststoffs vergrößert und der Gasaustausch durch den Hohlkörper oder den zumindest einen Kunststoff verbessert.

Ferner kann vorgesehen sein, dass der Gaseinleitungsschlauch und der Gasausleitungsschlauch bereichsweise koaxial zueinander angeordnet sind, wobei vorzugsweise beide gemeinsam durch eine Durchführung aus dem Hohlkörper geführt sind.

Dadurch gibt es nur einen Zugang zum Hohlkörper und nur einen gemeinsamen Durchgang zur Behandlungssituation, wodurch die Gefahr der mikrobiellen Verkeimung am Übergang des koaxialen Gaseinleitungsschlauchs und Gasausleitungsschlauchs von der Außenseite des Organismus zur Innenseite vermindert wird. Alternativ zu koaxialen Schläuchen ist es möglich, einen Schlauch zu nutzen, der durch eine Trennwand in zwei Schläuche geteilt ist, beispielsweise in zwei halbmondförmige beziehungsweise halbkreisförmige Schläuche geteilt ist.

Es kann auch vorgesehen sein, dass der Gaseinleitungsschlauch und der Gasausleitungsschlauch miteinander verbunden sind, wobei bevorzugt der Gaseinleitungsschlauch und der Gasausleitungsschlauch einen gemeinsamen Außenschlauch aufweisen und wobei besonders bevorzugt der gemeinsame Außenschlauch durch eine Trennwand unterteilt ist, die den Gaseinleitungsschlauch auf einer Seite begrenzt und den Gasausleitungsschlauch auf der anderen Seite begrenzt.

Ebenfalls besonders bevorzugt unterteilt eine Trennwand den gemeinsamen Außenschlauch in zwei im Querschnitt halbmondförmige oder halbkreisförmige Schläuche, die den Gaseinleitungsschlauch und den Gasausleitungsschlauch bilden.

Auch hierdurch wird die Gefahr für eine mikrobielle Verkeimung vermindert.

Gemäß einer bevorzugten Weiterbildung der vorliegenden Erfindung kann auch vorgesehen sein, dass in dem Gaseinleitungsschlauch und/oder dem Gasausleitungsschlauch ein keimundurchlässiger aber für Gase durchlässiger Sterilfilter angeordnet ist.

Hierdurch wird die Gefahr einer Infektion des behandelten Patienten und des behandelnden Personals reduziert.

Des Weiteren ist vorgesehen, dass in dem Gaseinleitungsschlauch und/oder dem Gasausleitungsschlauch das Einwegventil und das Überdruckventil angeordnet sind, wobei vorzugsweise der Gaseinleitungsschlauch und/oder der Gasausleitungsschlauch mit dem Ventil absperrbar ist.

Dadurch kann der expandierte Zustand (der aufgeblasene Zustand) des Hohlkörpers gehalten und gegebenenfalls auch bei Entfernung der Spülgasversorgung beziehungsweise der Spülflüssigkeitsversorgung erhalten bleiben.

Bevorzugt kann ferner vorgesehen sein, dass der Sterilfilter in dem Gaseinleitungsschlauch oder dem Gasausleitungsschlauch in Strömungsrichtung hinter dem Einwegventil beziehungsweise dem Überdruckventil angeordnet ist oder die Sterilfilter in dem Gaseinleitungsschlauch und in dem Gasausleitungsschlauch hinter dem Einwegventil beziehungsweise dem Überdruckventilangeordnet sind.

Hiermit kann ebenfalls die Gefahr einer Infektion des behandelten Patienten und des behandelnden Personals reduziert werden.

Des Weiteren ist vorgesehen, dass das Einwegventil im Gaseinleitungsschlauch angeordnet ist.

Dadurch wird ein automatischer gasdichter Verschluss des Gaseinleitungsschlauchs bei Abtrennung der Spülgasversorgung beziehungsweise der Spülflüssigkeitsversorgung erreicht, ohne dass manuell ein Ventil bedient werden muss. Die Anwendungssicherheit des medizinischen Platzhalters wird dadurch deutlich verbessert. Zudem kann so bei einem Expandieren des Hohlkörpers mit Überdruck verhindert werden, dass der Druck aus dem Hohlkörper durch den Gaseinleitungsschlauch entweicht.

Es ist vorgesehen, dass das Überdruckventil im Gasausleitungsschlauch angeordnet ist, wobei vorzugsweise der Öffnungsdruck des Überdruckventils einstellbar ist.

Dadurch kann der expandierte Zustand des Hohlkörpers sicher erreicht werden.

Es kann auch vorgesehen sein, dass der Gaseinleitungsschlauch und der Gasausleitungsschlauch aus einem für Sauerstoff und Kohlendioxid nicht durchlässigen Material bestehen, vorzugsweise aus einem für Sauerstoff und Kohlendioxid nicht durchlässigen Kunststoff bestehen.

Hierdurch wird verhindert, dass der Sauerstoff zu früh aus dem Spülgas beziehungsweise der Spülflüssigkeit austritt. Zudem können der Gaseinleitungsschlauch und der Gasausleitungsschlauch so aus einem kostengünstigeren Kunststoff gefertigt werden.

Ferner kann vorgesehen sein, dass der Hohlkörper oder der zumindest eine Kunststoff des Hohlkörpers mindestens einen antiseptischen Wirkstoff enthält oder mit mindestens einem antiseptischen Wirkstoff beschichtet ist.

Hierdurch können die Oberfläche des medizinischen Platzhalters und die Umgebung des medizinischen Platzhalters im Körper des Patienten mit dem mindestens einen antiseptischen Wirkstoff desinfiziert werden. Hierdurch werden Komplikationen bei der Behandlung vermieden.

Des Weiteren kann vorgesehen sein, dass in zumindest einer Wandung des Hohlkörpers oder des zumindest einen Kunststoffs des Hohlkörpers Durchführungen angeordnet sind, die in Richtung der Außenseite des Hohlkörpers weisende Öffnungen aufweisen und die zumindest einen Kanal mit der Umgebung des Hohlkörpers für Flüssigkeiten und Gase durchlässig verbinden, wobei der zumindest eine Kanal zum Einleiten von Flüssigkeiten in den Hohlkörper zu den Durchführungen vorgesehen ist, wobei vorzugsweise der zumindest eine Kanal mit einem Zuleitungsschlauch für pharmazeutische Wirkstofflösungen verbunden ist, wobei besonders bevorzugt an dem Zuleitungsschlauch ein Anschlussadapter, insbesondere ein Luer-Lock-Adapter angeordnet ist.

Dadurch ist es möglich, die Durchführungen mit dem Zuleitungsschlauch zu verbinden, durch den antiseptische oder antibiotische Lösungen in die Durchführungen gepresst werden können. Durch die Durchführungen ist ein antimikrobieller Schutz der Oberfläche des Hohlkörpers möglich. Es ist auch möglich, in die Durchführungen flüssige oder gelartige antibiotische oder antiseptische pharmazeutische Zubereitungen einzubringen, die durch Diffusion in die Umgebung migrieren können und so zur Behandlung des Patienten im Bereich des medizinischen Platzhalters verwendet werden können.

Ferner kann vorgesehen sein, dass der Hohlkörper im nicht aufgeblasenen Zustand an dem Gaseinleitungsschlauch und/oder an dem Gasausleitungsschlauch anliegt, wobei vorzugsweise der Hohlkörper oder der zumindest eine Kunststoff nach Art eines Ballons ausblasbar ist.

Hierdurch ist der medizinische Platzhalter im nicht aufgeblasenen Zustand (im nicht expandierten Zustand) kompakt und kann so leichter in den Knochendefekt eingebracht und aus dem Knochendefekt wieder entfernt werden.

Die der vorliegenden Erfindung zugrundeliegenden Aufgaben werden auch gelöst durch ein Verfahren zum Begasen einer Oberfläche eines medizinischen Platzhalters, insbesondere eines erfindungsgemäßen medizinischen Platzhalters, wobei das Verfahren nicht zur medizinischen Behandlung eines menschlichen oder tierischen Körpers durchgeführt wird, gekennzeichnet durch die folgenden Schritte:
A) Expandieren eines Hohlkörpers durch Einleiten einer Spülflüssigkeit oder eines Spülgases enthaltend Sauerstoff in einen Innenraum des Hohlkörpers, so dass der Innenraum des Hohlkörpers vergrößert wird;
B) Abgeben von gasförmigem Sauerstoff aus der Spülflüssigkeit oder dem Spülgas durch einen den Innenraum begrenzenden Kunststoff hindurch an die Umgebung des Hohlkörpers;
C) Aufnehmen von gasförmigem Kohlendioxid aus der Umgebung des Hohlkörpers durch den den Innenraum begrenzenden Kunststoff hindurch in die Spülflüssigkeit oder das Spülgas;
D) Hindurchleiten der Spülflüssigkeit oder des Spülgases durch den Innenraum des Hohlkörpers und Ableiten der Spülflüssigkeit oder des Spülgases aus dem Innenraum.

Die Schritte B) und C) laufen vorzugsweise gleichzeitig ab. Zudem erfolgt der Gasaustausch in den Schritten B) und C) bevorzugt auch schon während Schritt A) ab und die ganze Zeit während Schritt D).

Als Spülgas können Luft oder Sauerstoff verwendet werden. Im Rahmen der Erfindung ist es auch, wenn anstelle von Luft oder Sauerstoff als das Spülgas mit Sauerstoff gesättigte Spülflüssigkeiten, wie zum Beispiel physiologische Kochsalzlösung, Ringer-Lösung oder Ringer-Lactat-Lösung, in den expandierbaren medizinischen Platzhalter eingebracht werden, insbesondere durch den Gaseinleitungsschlauch. Es ist weiterhin auch möglich, als Sauerstoffträger perfluoriertes Decalin oder andere perfluorierte Spülflüssigkeiten zu verwenden, in denen Sauerstoff löslich ist. Durch den Gasausleitungsschlauch können diese Spülflüssigkeiten aus dem Hohlkörper ausgeleitet werden.

Es kann bevorzugt vorgesehen sein, dass das Verfahren außerhalb eines menschlichen oder eines tierischen Körpers durchgeführt wird.

Es kann auch vorgesehen sein, dass das erfindungsgemäße Verfahren nicht von einem Arzt oder einem Mediziner durchgeführt wird.

Bevorzugt kann ferner vorgesehen sein, dass vor Schritt A) der Hohlkörper in einen Hohlraum eingeführt wird, wobei während Schritt A) die Form des aufgeblasenen Hohlkörpers sich zumindest bereichsweise an die Form des Hohlraums anpasst und während der Schritte B) und C) zumindest bereichsweise an dem Hohlraum anliegt.

Der Hohlraum ist bevorzugt keine Kavität eines menschlichen oder tierischen Körpers.

Durch das Einführen in den Hohlraum werden die Vorteile des Verfahrens voll zu Geltung gebracht. Der Gasaustausch zwischen dem Hohlraum und dem Innenraum des Hohlkörpers bewirkt die erfindungsgemäßen Vorteile.

Des Weiteren kann vorgesehen sein, dass in Schritt A) der Hohlkörper aufgeblasen wird, bis ein Mindestdruck erreicht wird, wobei sich bei Erreichen des Mindestdrucks ein Überdruckventil öffnet, durch das die Spülflüssigkeit oder das Spülgas aus dem Innenraum des Hohlkörpers während Schritt D) abgeleitet wird.

Hierdurch kann die Form des Hohlkörpers an eine umgebende Form angepasst werden, so dass die Umgebung des Hohlkörpers mit dem Hohlkörper mechanisch stabilisiert werden kann.

Der Erfindung liegt die überraschende Erkenntnis zugrunde, dass es durch den expandierbaren medizinischen Platzhalter gelingt, den Aufbau einer Bindegewebsmembran zu unterstützen, indem der medizinische Platzhalter an die Form des Knochendefekts anpassbar ist und indem eine Versorgung der Bindegewebsmembran mit Sauerstoff erfolgt und eine Übersäuerung der Bindegewebsmembran durch Kohlendioxid beziehungsweise Kohlensäure vermieden wird. Gleichzeitig kann der medizinische Platzhalter nach einer Volumenkontraktion leichter durch einen kleinen Schnitt in der Bindegewebsmembran wieder entfernt werden. Aus diesen Gründen kann eine schnellere und wirkungsvollere Ossifikation gelingen, so dass der Heilungserfolg schneller eintreten kann. Der besondere Vorteil ist dabei insbesondere auch darin zu sehen, dass die vom Blutkreislauf abgewandte Innenseite der Bindegewebsschicht, die normalerweise vom Blutkreislauf nur schlecht mit Sauerstoff versorgt werden kann, mit dem erfindungsgemäßen medizinischen Platzhalter besser versorgt werden kann, da dieser direkt an die Bindegewebsschicht angrenzt. Mit herkömmlichen massiven Platzhaltern ist dies nicht möglich.

Der Vorteil des erfindungsgemäßen medizinischen Platzhalters besteht somit darin, dass der Hohlkörper durch Expandieren (beispielsweise durch Aufblasen) in seiner Gestalt dem auszufüllenden Knochendefekt angepasst werden kann. Weiterhin ist vorteilhaft, dass nach Beendigung der Implantationsphase der Hohlkörper durch Anlegen von Unterdruck sein Volumen verringern kann und dadurch die Entfernung des Hohlkörpers durch eine kleine Öffnung in der gebildeten Bindegewebsmembran erfolgen kann, so dass die Bindegewebsmembran weitgehend unbeschädigt bleibt. Ein wesentlicher Vorteil des erfindungsgemäßen medizinischen Platzhalters ist die Versorgung der Zellen der sich ausbildenden Bindegewebsmembran mit Sauerstoff und des Abtransports des anfallenden Kohlendioxids. Dadurch wird ein Sauerstoffmangel auch bei der Weichgewebedeckung mit einem vorgeschädigten Weichgewebe ermöglicht. Der Abtransport des Kohlendioxids verhindert eine Versäuerung der Zellen der sich ausbildenden Bindegewebsmembran. Diese beiden Prozesse unterstützen die Ausbildung einer vitalen Bindegewebsmembran.

Wesentlich für die Ossifikation ist, dass die vaskularisierte Bindegewebsmembran ausreichend vital ist und durch das überdeckende Weichgewebe mit Sauerstoff und Nährstoffen versorgt wird.

Es wurde im Rahmen der vorliegenden Erfindung erkannt, dass während der Implantationsdauer herkömmlicher Knochenzementspacer die sich ausbildende Bindegewebsmembran im Wesentlichen durch das überdeckende Weichgewebe mit Sauerstoff und Nährstoffen versorgt wird. Der Abtransport des bei dem Stoffwechsel anfallenden Kohlendioxids erfolgt ebenfalls über das überdeckende Weichgewebe. Wenn das Weichgewebe nur noch in geringer Stärke vorhanden ist oder durch die Knochendefektentstehung eine Vorschädigung des Weichgewebes stattgefunden hat, ist eine verzögerte Ausbildung der Bindegewebsmembran infolge einer unzureichenden Sauerstoff- und Nährstoffversorgung als auch dem ungenügenden Abtransport von Kohlendioxid zu erwarten. Wünschenswert ist es, auch bei geringer Weichteildeckung oder auch bei Weichteildeckung mit vorgeschädigten Weichgewebe, sicher eine Bindegewebsmembran zu erzeugen. Dazu sollten die Zellen, die sich unmittelbar an der Spaceroberfläche befinden optimal mit Sauerstoff versorgt werden. Weiterhin soll das anfallende Kohlendioxid entfernt werden, um eine Absenkung des lokalen pH-Wertes zu vermeiden. Eine Absenkung des pH-Wertes kann eine Zellschädigung hervorrufen.

Ein beispielhafter erfindungsgemäßer expandierbarer medizinischer Platzhalter kann beispielsweise aufweisen:
a) mindestens einen expandierbaren Kunststoffhohlkörper mit einem Innenraum,
b) mindestens einen Gaseinleitungsschlauch, der mit dem Innenraum des Kunststoffhohlkörpers gasdurchlässig verbunden ist,
c) mindestens einem Gasausleitungsschlauch, der mit dem Innenraum des Kunststoffhohlkörpers gasdurchlässig verbunden ist, und
d) wobei der Kunststoffhohlkörper einen Permeabilitätskoeffizienten für Sauerstoff von gleich/größer 0,5 cm³/(m² * d * bar) und einen Permeabilitätskoeffizienten für Kohlendioxid von gleich/größer 0,5 cm³/(m² * d * bar) besitzt.

Im Folgenden werden sechs weitere Ausführungsbeispiele der Erfindung anhand von neunzehn schematisch dargestellten Figuren erläutert, ohne jedoch dabei die Erfindung zu beschränken. Dabei zeigt:
Figur 1: eine schematische Aufsicht auf einen ersten beispielhaften erfindungsgemäßen medizinischen Platzhalter im komprimierten Zustand;
Figur 2: eine schematische Querschnittansicht auf den ersten beispielhaften medizinischen Platzhalter nach Figur 1 im komprimierten Zustand, wobei die Schnittebene in der Bildebene der Figur 1 liegt;
Figur 3: eine schematische Aufsicht auf den ersten beispielhaften medizinischen Platzhalter nach den Figuren 1 und 2 im expandierten Zustand;
Figur 4: eine schematische Querschnittansicht auf den medizinischen Platzhalter nach den Figuren 1 bis 3 im expandierten Zustand, wobei die Schnittebene in der Bildebene der Figur 3 liegt;
Figur 5: die schematische Querschnittansicht nach Figur 4, wobei die Strömungsverhältnisse mit Pfeilen eingezeichnet sind;
Figur 6: eine schematische Aufsicht auf einen zweiten beispielhaften erfindungsgemäßen medizinischen Platzhalter im komprimierten Zustand;
Figur 7: eine schematische Querschnittansicht auf den zweiten beispielhaften medizinischen Platzhalter nach Figur 6 im komprimierten Zustand, wobei die Schnittebene in der Bildebene der Figur 6 liegt;
Figur 8: eine schematische Querschnittansicht auf den zweiten beispielhaften medizinischen Platzhalter nach den Figuren 6 und 7 im expandierten Zustand;
Figur 9: eine schematische perspektivische Teilquerschnittansicht des zweiten beispielhaften medizinischen Platzhalters nach den Figuren 6 bis 8 im expandierten Zustand, wobei die Schnittebene in der Bildebene der Figur 6 liegt;
Figur 10: eine schematische Querschnittansicht eines dritten beispielhaften erfindungsgemäßen medizinischen Platzhalters im komprimierten Zustand;
Figur 11: eine schematische Querschnittansicht des dritten beispielhaften medizinischen Platzhalter nach Figur 10 im expandierten Zustand;
Figur 12: eine schematische perspektivische Teilquerschnittansicht des dritten beispielhaften medizinischen Platzhalters nach den Figuren 10 und 11 im expandierten Zustand;
Figur 13: eine schematische Querschnittansicht eines vierten beispielhaften erfindungsgemäßen medizinischen Platzhalters im komprimierten Zustand;
Figur 14: eine schematische Querschnittansicht des vierten beispielhaften medizinischen Platzhalter nach Figur 13 im expandierten Zustand;
Figur 15: eine schematische perspektivische Teilquerschnittansicht des vierten beispielhaften medizinischen Platzhalters nach den Figuren 13 und 14 im expandierten Zustand;
Figur 16: eine schematische Querschnittansicht eines fünften beispielhaften erfindungsgemäßen medizinischen Platzhalters im komprimierten Zustand;
Figur 17: eine schematische Querschnittansicht des fünften beispielhaften medizinischen Platzhalter nach Figur 16 im expandierten Zustand;
Figur 18: eine schematische Querschnittansicht eines sechsten beispielhaften erfindungsgemäßen medizinischen Platzhalters im komprimierten Zustand; und
Figur 19: eine schematische Querschnittansicht des sechsten beispielhaften medizinischen Platzhalter nach Figur 18 im expandierten Zustand.

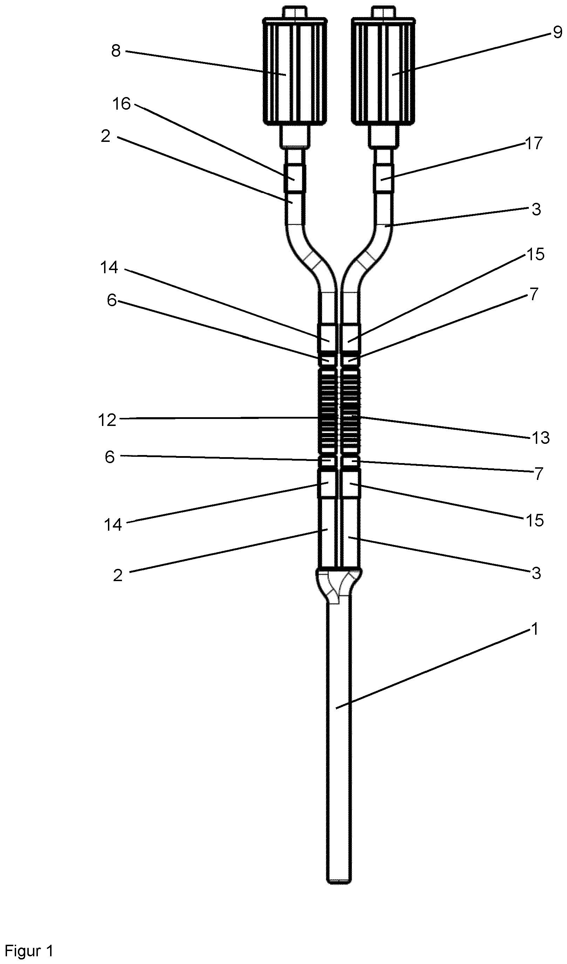

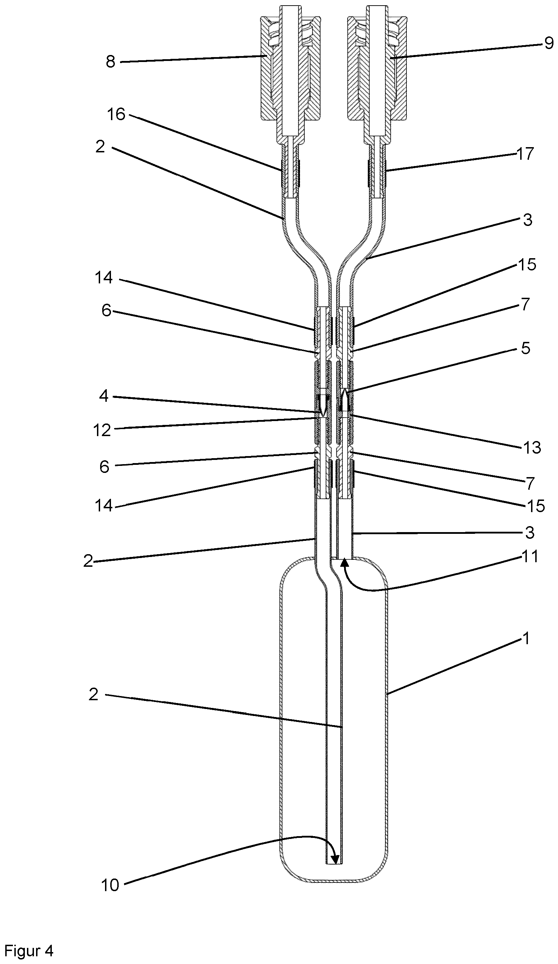

In den Figuren 1 bis 17 ist die Vorderseite des jeweiligen medizinischer Platzhalters nach unten und die Rückseite nach oben ausgerichtet.

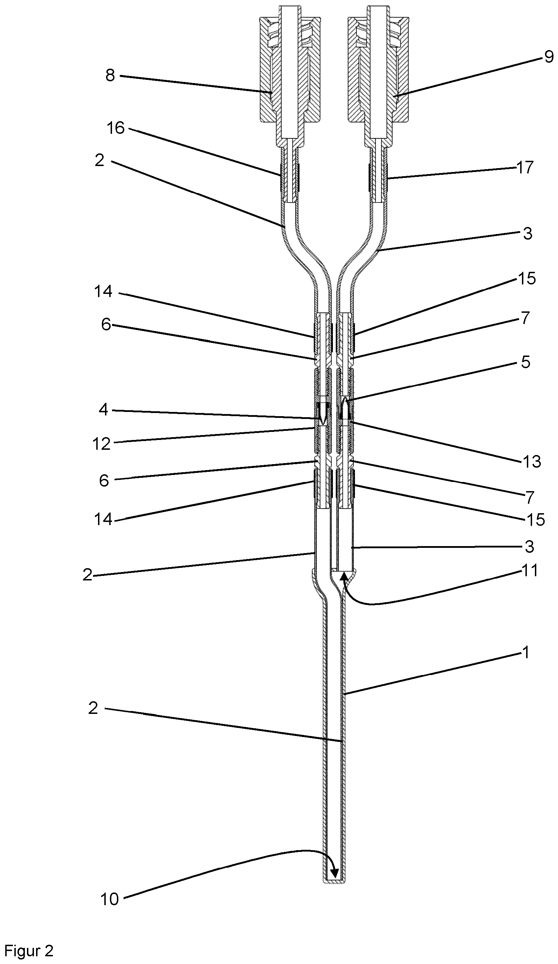

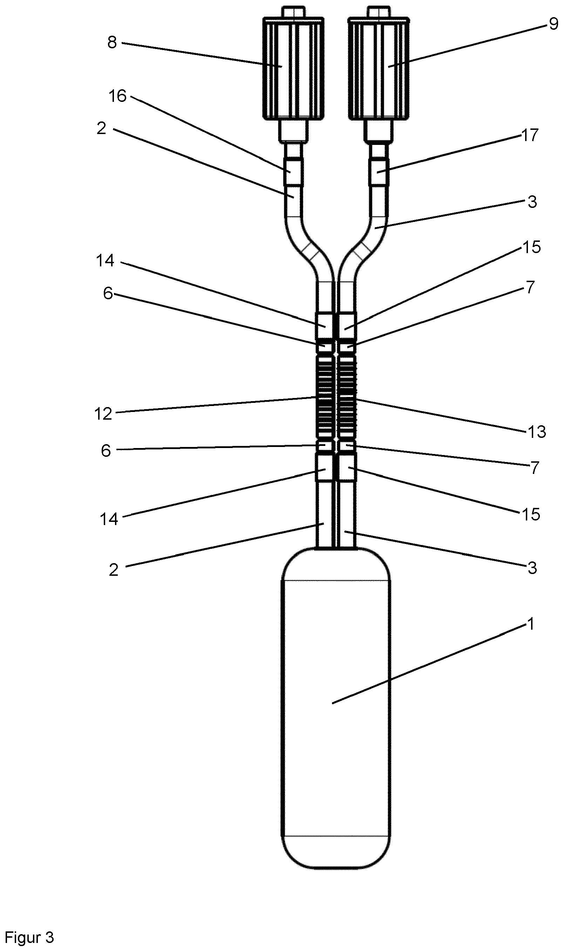

In den Figuren 1 bis 5 sind Abbildungen eines ersten erfindungsgemäßen medizinischen Platzhalters gezeigt. Die Figuren 1 und 2 zeigen den Platzhalter im komprimierten Zustand und die Figuren 3 bis 5 zeigen den Platzhalter im expandierten Zustand.

Der erste erfindungsgemäße medizinische Platzhalter kann einen Hohlkörper 1 aus einem elastisch oder plastisch verformbaren Kunststoff aufweisen. Der Hohlkörper 1 kann expandierbar sein, indem er mit einem Spülgas aufgeblasen wird oder indem eine Spülflüssigkeit mit einem Druck in den Hohlkörper 1 eingedrückt wird. Der Hohlkörper 1 kann beispielsweise aus einem Gummi bestehen.

Das für den Hohlkörper 1 verwendete Material kann für gasförmigen molekularen Sauerstoff und für gasförmiges Kohlendioxid durchlässig sein. Der Hohlkörper 1 beziehungsweise das Material, aus dem der Hohlkörper 1 gefertigt ist, kann hierzu einen Permeabilitätskoeffizienten für Sauerstoff von größer oder gleich 0,5 cm³/(m²*d*bar) und einen Permeabilitätskoeffizienten für Kohlendioxid von größer oder gleich 0,5 cm³/(m²*d*bar) aufweisen. Der Permeabilitätskoeffizient wird nach DIN 53380-4 (11/2006) bestimmt.

Zum Einleiten eines Spülfluids (eines Spülgases oder einer Spülflüssigkeit) kann ein Innenraum des Hohlkörpers 1 mit einem Gaseinleitungsschlauch 2 aus Kunststoff verbunden sein. Zum Ableiten des Spülfluids (des Spülgases oder der Spülflüssigkeit) aus dem Hohlkörper 1 heraus kann der Innenraum des Hohlkörpers 1 mit einem Gasausleitungsschlauch 3 aus Kunststoff verbunden sein. Der Gaseinleitungsschlauch 2 und der Gasausleitungsschlauch 3 können zumindest bereichsweise flexibel und beweglich sein.

In dem Gaseinleitungsschlauch 2 kann ein Ventil 4 in Form eines Lippenventils angeordnet sein, das eine Strömung des Spülfluids in Richtung des Hohlkörpers 1 ermöglicht aber eine Strömung des Spülfluids in der Richtung vom Hohlkörper 1 weg verhindert. In dem Gasausleitungsschlauch 3 kann ein Ventil 5 in Form eines Lippenventils angeordnet sein, das eine Strömung des Spülfluids in Richtung des Hohlkörpers 1 verhindert aber eine Strömung des Spülfluids in der Richtung vom Hohlkörper 1 weg erlaubt. Das Ventil 5 kann dazu ausgelegt sein, dass es ab einem Mindestdruck des Spülfluids öffnet. Bevorzugt ist der Mindestdruck am Ventil 5 einstellbar. Der Mindestdruck kann dabei so gewählt werden, dass der Druck des Spülfluids dazu ausreicht, den Hohlkörper 1 vom komprimierten Zustand (siehe Figuren 1 und 2) in den expandierten Zustand zu überführen (siehe Figuren 3 bis 5).

Die Ventile 4, 5 können über Verbindungshülsen 6, 7 mit dem Gaseinleitungsschlauch 2 und dem Gasausleitungsschlauch 3 verbunden sein. Hierzu kann der Gaseinleitungsschlauch 2 auf die Verbindungshülse 6 gesteckt sein und dort mit einer Krimphülse 14 befestigt sein. Ebenso kann der Gasausleitungsschlauch 3 auf die Verbindungshülse 7 gesteckt sein und dort mit einer Krimphülse 15 befestigt sein.

Der Gaseinleitungsschlauch 2 kann an seiner Rückseite in einem Adapter 8 in Form eines Luer-Lock-Adapters enden. Ebenso kann der Gasausleitungsschlauch 3 an seiner Rückseite in einem Adapter 9 in Form eines Luer-Lock-Adapters enden. Durch die Adapter 8, 9 kann das Spülfluid eingespeist und abgeleitet werden.

Der Gaseinleitungsschlauch 2 kann über eine Einströmöffnung 10 in den vorderen Teil des Innenraums des Hohlkörpers 1 münden, während der Gasausleitungsschlauch 3 über eine Ausströmöffnung 11 an der gegenüberliegenden Rückseite des Innenraums des Hohlkörpers 1 mit dem Innenraum des Hohlkörpers 1 verbunden sein kann. Hierdurch wird sichergestellt, dass das Spülfluid entlang der Oberfläche der Wandung des gesamten Hohlkörpers 1 strömen kann und dadurch ein Gasaustausch von Sauerstoff und Kohlendioxid durch die Wandung auf der gesamten Länge des Hohlkörpers 1 erfolgen kann.

Das Ventil 4 kann ein Ventilgehäuse 12 aufweisen. Das Ventilgehäuse 12 kann über Innengewinde mit Außengewinden der Verbindungshülsen 6 verbunden sein. Das Ventil 5 kann ein Ventilgehäuse 13 aufweisen. Das Ventilgehäuse 13 kann über Innengewinde mit Außengewinden der Verbindungshülsen 7 verbunden sein. Alle Verbindungen können gasdicht und Druckdicht ausgeführt sein.

Der Gaseinleitungsschlauch 2 kann auf einen Stutzen des Adapters 8 aufgesteckt sein und dort mit einer Krimphülse 16 druckdicht und gasdicht befestigt werden. Der Gasausleitungsschlauch 3 kann auf einen Stutzen des Adapters 9 aufgesteckt sein und dort mit einer Krimphülse 17 druckdicht und gasdicht befestigt werden.

Der Hohlkörper 1 kann im komprimierten Zustand auch durch eine kleine Öffnung in einen Hohlraum eingebracht werden. Dort kann der Hohlkörper 1 expandiert werden und so die äußere Form des Hohlkörpers 1 an den Hohlraum angepasst werden. Der expandierte Hohlkörper 1, beziehungsweise der Platzhalter im expandierten Zustand, kann so den Hohlraum mechanisch stützen und stabilisieren. Wenn der Platzhalter nicht mehr benötigt wird, kann der Hohlkörper 1 wieder in den komprimierten Zustand überführt werden, beispielsweise indem er evakuiert wird. Der Hohlkörper 1 kann dann wieder durch eine enge Öffnung aus dem Hohlraum entfernt werden. Insbesondere wenn der Hohlraum eine Kavität in einem Knochen ist, kann dieser Knochendefekt so schonend behandelt werden.

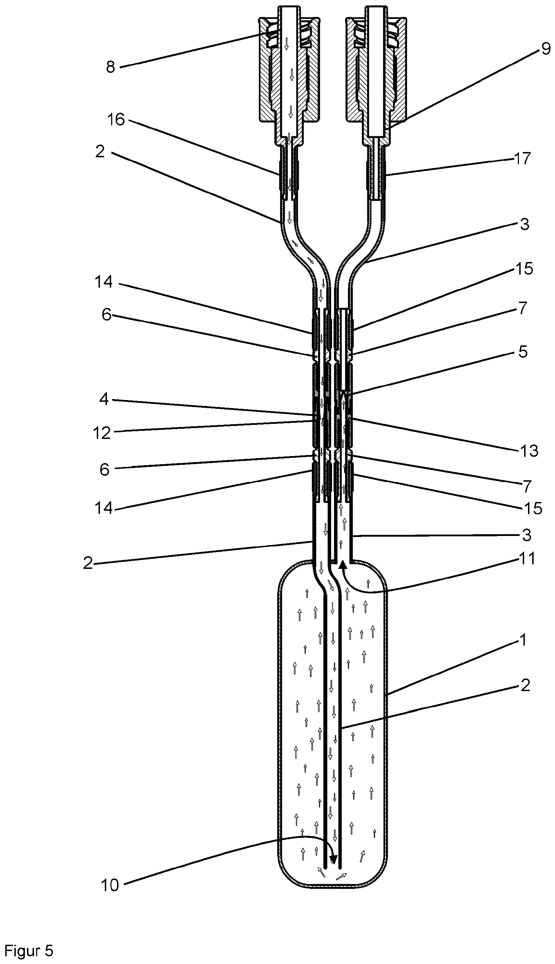

Im Betrieb kann das Spülfluid durch den Adapter 8 in den medizinischen Platzhalter eingespeist werden. Das Spülfluid kann durch den Gaseinleitungsschlauch 2 strömen und bei ausreichendem Druck das Ventil 4 öffnen. Dann kann das Spülfluid durch die Einströmöffnung 10 in den Hohlkörper 1 und durch den Hohlkörper 1 fließen. Das Spülfluid kann durch den Gasausleitungsschlauch 3 bis zum zunächst geschlossenen Ventil 5 strömen. Dabei kann sich im Inneren des Hohlkörpers 1 ein Druck aufbauen, mit dem der Hohlkörper 1 in den expandierten Zustand überführt wird (siehe Figur 5). Sobald der Druck am Ventil 5 im Gasausleitungsschlauch 3 ausreicht, öffnet sich das Ventil 5 und das Spülfluid fließt durch den Gasausleitungsschlauch 3 und den Adapter 9 ab.

In dem Spülfluid ist Sauerstoff enthalten. Das Spülfluid kann durch die Wandung des Hohlkörpers 1 Sauerstoff an die Umgebung des Hohlkörpers 1 abgeben. Gleichzeitig kann das strömende Spülfluid Kohlendioxid aus der Umgebung des Hohlkörpers 1, das durch die Wandung des Hohlkörpers 1 in den Innenraum diffundiert, aufnehmen, und aus dem medizinischen Platzhalter durch den Adapter 9 ableiten. Hierdurch kann die Umgebung des Hohlkörpers 1 mit Sauerstoff versorgt werden und durch die Aufnahme von Kohlendioxid eine Übersäuerung der Umgebung des Hohlkörpers 1 verhindert werden.

In dem Gaseinleitungsschlauch 2 und/oder in dem Gasausleitungsschlauch 3 können bevorzugt keimundurchlässige aber für Gase durchlässige Sterilfilter (nicht gezeigt) angeordnet sein. Das Spülfluid kann mit dem Sterilfilter von Keimen befreit werden, die ansonsten in den Hohlkörper 1 gelangen könnten und/oder die vom Hohlkörper 1 durch den Adapter 9 abgeleitet werden könnten. Hierdurch wird die Gefahr einer Infektion des behandelten Patienten und des behandelnden Personals reduziert.

Bevorzugt kann der Sterilfilter in dem Gaseinleitungsschlauch 2 oder dem Gasausleitungsschlauch 3 in Strömungsrichtung hinter dem Ventil 4 oder dem Ventil 5 angeordnet sein oder die Sterilfilter in dem Gaseinleitungsschlauch 2 und in dem Gasausleitungsschlauch 3 hinter den Ventilen 4, 5 angeordnet sein.

Es kann auch vorgesehen sein, dass der Hohlkörper 1 sowie die angrenzenden Bereiche der Schläuche 2, 3 mit einer antiseptischen Substanz beschichtet ist oder eine lösbare antiseptische Substanz in dem Material des Hohlkörpers 1 enthalten ist, um eine Infektion zu verhindern.

Zur Behandlung der Umgebung des Hohlkörpers 1 kann zudem vorgesehen sein, dass in zumindest einer Wandung des Hohlkörpers 1 oder des zumindest einen Kunststoffs des Hohlkörpers 1 Durchführungen (nicht gezeigt) angeordnet sind, die in Richtung der Außenseite des Hohlkörpers 1 weisende Öffnungen aufweisen, die zumindest einen Kanal (nicht gezeigt) mit der Umgebung des Hohlkörpers 1 für Flüssigkeiten und Gase durchlässig verbinden. Der zumindest eine Kanal kann zum Einleiten von Flüssigkeiten in den Hohlkörper 1 zu den Durchführungen vorgesehen sein. Ferner kann der zumindest eine Kanal mit einem Zuleitungsschlauch (nicht gezeigt) für pharmazeutische Wirkstofflösungen verbunden sein. Dadurch kann eine pharmazeutische Wirkstofflösung an der Oberfläche des Hohlkörpers 1 abgegeben werden.

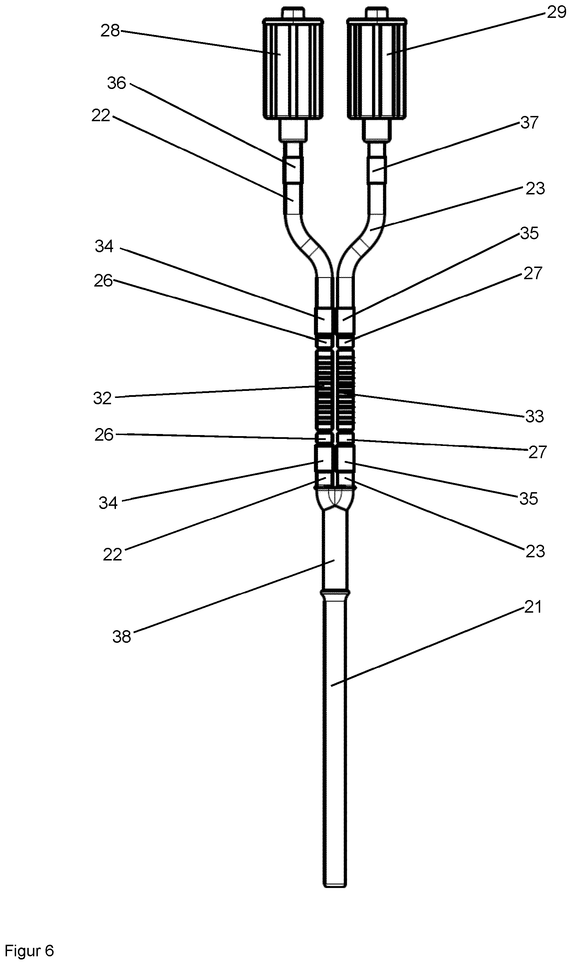

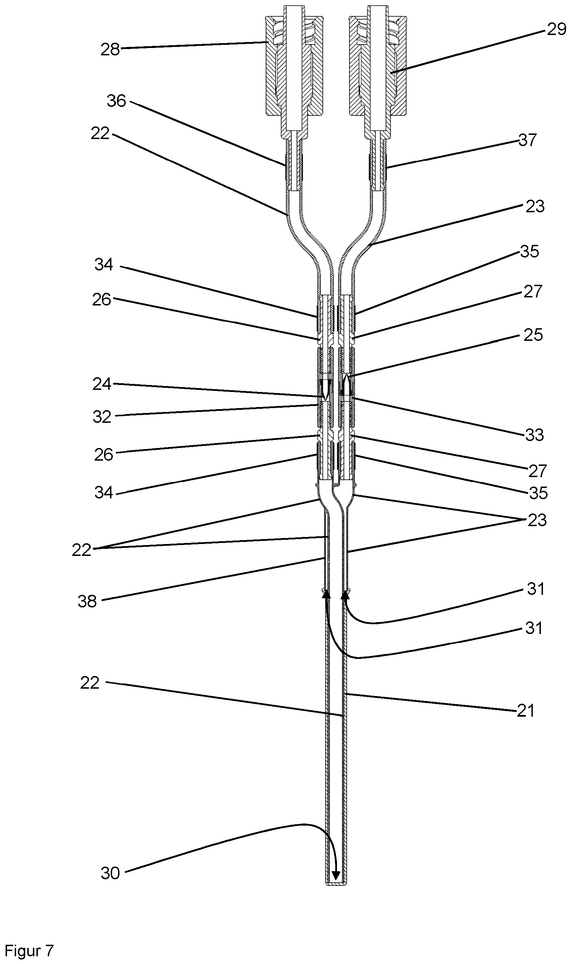

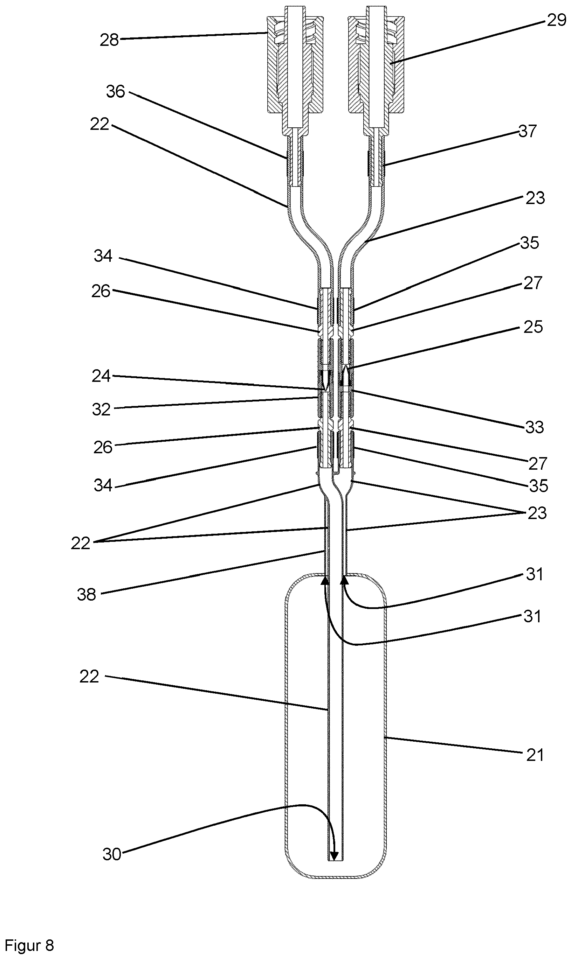

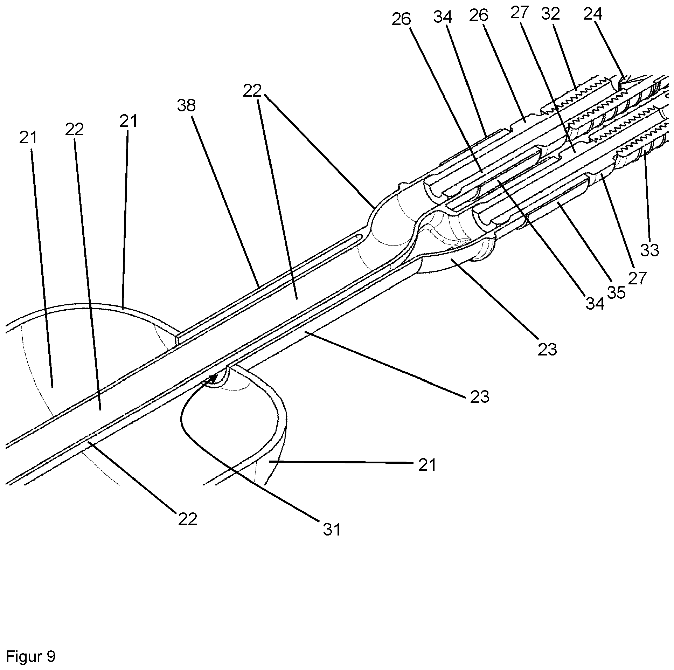

In den Figuren 6 bis 9 sind Abbildungen eines zweiten erfindungsgemäßen medizinischen Platzhalters gezeigt. Die Figuren 6 und 7 zeigen den Platzhalter im komprimierten Zustand und die Figuren 8 und 9 zeigen den Platzhalter im expandierten Zustand.

Der zweite erfindungsgemäße medizinische Platzhalter kann einen Hohlkörper 21 aus einem elastisch oder plastisch verformbaren Kunststoff aufweisen. Der Hohlkörper 21 kann expandierbar sein, indem er mit einem Spülgas aufgeblasen wird oder indem eine Spülflüssigkeit mit einem Druck in den Hohlkörper 21 eingedrückt wird. Der Hohlkörper 21 kann beispielsweise aus einem Gummi bestehen.

Das für den Hohlkörper 21 verwendete Material kann für gasförmigen molekularen Sauerstoff und für gasförmiges Kohlendioxid durchlässig sein. Der Hohlkörper 21 beziehungsweise das Material, aus dem der Hohlkörper 21 gefertigt ist, kann hierzu einen Permeabilitätskoeffizienten für Sauerstoff von größer oder gleich 0,5 cm³/(m²*d*bar) und einen Permeabilitätskoeffizienten für Kohlendioxid von größer oder gleich 0,5 cm³/(m²*d*bar) aufweisen. Der Permeabilitätskoeffizient wird nach DIN 53380-4 (11/2006) bestimmt.

Zum Einleiten eines Spülfluids (eines Spülgases oder einer Spülflüssigkeit) kann ein Innenraum des Hohlkörpers 21 mit einem Gaseinleitungsschlauch 22 aus Kunststoff verbunden sein. Zum Ableiten des Spülfluids (des Spülgases oder der Spülflüssigkeit) aus dem Hohlkörper 21 heraus kann der Innenraum des Hohlkörpers 21 mit einem Gasausleitungsschlauch 23 aus Kunststoff verbunden sein. Der Gaseinleitungsschlauch 22 und der Gasausleitungsschlauch 23 können zumindest bereichsweise flexibel und beweglich sein. In dem Gaseinleitungsschlauch 22 kann ein Ventil 24 in Form eines Lippenventils angeordnet sein, das eine Strömung des Spülfluids in Richtung des Hohlkörpers 21 ermöglicht aber eine Strömung des Spülfluids in der Richtung vom Hohlkörper 21 weg verhindert. In dem Gasausleitungsschlauch 23 kann ein Ventil 25 in Form eines Lippenventils angeordnet sein, das eine Strömung des Spülfluids in Richtung des Hohlkörpers 21 verhindert aber eine Strömung des Spülfluids in der Richtung vom Hohlkörper 21 weg erlaubt. Das Ventil 25 kann dazu ausgelegt sein, dass es ab einem Mindestdruck des Spülfluids öffnet. Bevorzugt ist der Mindestdruck am Ventil 25 einstellbar. Der Mindestdruck kann dabei so gewählt werden, dass der Druck des Spülfluids dazu ausreicht, den Hohlkörper 21 vom komprimierten Zustand (siehe Figuren 6 und 7) in den expandierten Zustand zu überführen (siehe Figuren 8 und 9).

Die Ventile 24, 25 können über Verbindungshülsen 26, 27 mit dem Gaseinleitungsschlauch 22 und dem Gasausleitungsschlauch 23 verbunden sein. Hierzu kann der Gaseinleitungsschlauch 22 auf die Verbindungshülse 26 gesteckt sein und dort mit einer Krimphülse 34 befestigt sein. Ebenso kann der Gasausleitungsschlauch 23 auf die Verbindungshülse 27 gesteckt sein und dort mit einer Krimphülse 35 befestigt sein.

Der Gaseinleitungsschlauch 22 kann an seiner Rückseite in einem Adapter 28 in Form eines Luer-Lock-Adapters enden. Ebenso kann der Gasausleitungsschlauch 23 an seiner Rückseite in einem Adapter 29 in Form eines Luer-Lock-Adapters enden. Durch die Adapter 28, 29 kann das Spülfluid eingespeist und abgeleitet werden.

Der Gaseinleitungsschlauch 22 kann über eine Einströmöffnung 30 in den vorderen Teil des Innenraums des Hohlkörpers 21 münden, während der Gasausleitungsschlauch 23 über eine Ausströmöffnung 31 an der gegenüberliegenden Rückseite des Innenraums des Hohlkörpers 21 mit dem Innenraum des Hohlkörpers 21 verbunden sein kann. Hierdurch wird sichergestellt, dass das Spülfluid entlang der Oberfläche der Wandung des gesamten Hohlkörpers 21 strömen kann und dadurch ein Gasaustausch von Sauerstoff und Kohlendioxid durch die Wandung auf der gesamten Länge des Hohlkörpers 21 erfolgen kann.

Das Ventil 24 kann ein Ventilgehäuse 32 aufweisen. Das Ventilgehäuse 32 kann über Innengewinde mit Außengewinden der Verbindungshülsen 26 verbunden sein. Das Ventil 25 kann ein Ventilgehäuse 33 aufweisen. Das Ventilgehäuse 33 kann über Innengewinde mit Außengewinden der Verbindungshülsen 27 verbunden sein. Alle Verbindungen können gasdicht und Druckdicht ausgeführt sein.

Der Gaseinleitungsschlauch 22 kann auf einen Stutzen des Adapters 28 aufgesteckt sein und dort mit einer Krimphülse 36 druckdicht und gasdicht befestigt werden. Der Gasausleitungsschlauch 23 kann auf einen Stutzen des Adapters 29 aufgesteckt sein und dort mit einer Krimphülse 37 druckdicht und gasdicht befestigt werden.

Der Gaseinleitungsschlauch 22 und der Gasausleitungsschlauch 23 sind bei dem zweiten Ausführungsbeispiel im Unterschied zum ersten Ausführungsbeispiel bereits vor dem Hohlkörper 21 zusammengeführt und bilden dort einen Koaxialschlauch 38. Dabei kann der Gasausleitungsschlauch 23 innerhalb des Gaseinleitungsschlauchs 22 angeordnet sein (wie in den Figuren 6 bis 9 gezeigt) oder der Gaseinleitungsschlauch 22 kann innerhalb des Gasausleitungsschlauchs 23 angeordnet sein. Der Vorteil ist, dass nur eine Verbindung vorhanden ist, so dass der Durchgang leichter zu schließen ist und die Gefahr für einen Durchtritt von Keimen reduziert ist. Zudem mündet die Ausströmöffnung 31 um den Gaseinleitungsschlauch 22 herum in den Innenraum des Hohlkörpers 21 (oder alternativ die Einströmöffnung 30 um den Gasausleitungsschlauch herum in den Innenraum des Hohlkörpers 21). Hierdurch kann die gesamte Oberfläche des Hohlkörpers 21 noch direkter und leichter von dem Spülfluid überstrichen werden und damit der Gasaustausch verbessert werden.

Im Betrieb kann das Spülfluid durch den Adapter 28 in den medizinischen Platzhalter eingespeist werden. Das Spülfluid kann durch den Gaseinleitungsschlauch 22 strömen und bei ausreichendem Druck das Ventil 24 öffnen. Dann kann das Spülfluid durch die Einströmöffnung 30 in den Hohlkörper 21 und durch den Hohlkörper 21 fließen. Das Spülfluid kann durch den Gasausleitungsschlauch 23 bis zum zunächst geschlossenen Ventil 25 strömen. Dabei kann sich im Inneren des Hohlkörpers 21 ein Druck aufbauen, mit dem der Hohlkörper 21 in den expandierten Zustand überführt wird. Sobald der Druck am Ventil 25 im Gasausleitungsschlauch 23 ausreicht, öffnet sich das Ventil 25 und das Spülfluid fließt durch den Gasausleitungsschlauch 23 und den Adapter 29 ab.

Der Hohlkörper 21 kann im komprimierten Zustand auch durch eine kleine Öffnung in einen Hohlraum eingebracht werden. Dort kann der Hohlkörper 21 expandiert werden und so die äußere Form des Hohlkörpers 21 an den Hohlraum angepasst werden. Der expandierte Hohlkörper 21, beziehungsweise der Platzhalter im expandierten Zustand, kann so den Hohlraum mechanisch stützen und stabilisieren. Wenn der Platzhalter nicht mehr benötigt wird, kann der Hohlkörper 21 wieder in den komprimierten Zustand überführt werden, beispielsweise indem er evakuiert wird. Der Hohlkörper 21 kann dann wieder durch eine enge Öffnung aus dem Hohlraum entfernt werden. Insbesondere wenn der Hohlraum eine Kavität in einem Knochen ist, kann dieser Knochendefekt so schonend behandelt werden.

In dem Spülfluid ist Sauerstoff enthalten. Das Spülfluid kann durch die Wandung des Hohlkörpers 21 Sauerstoff an die Umgebung des Hohlkörpers 21 abgeben. Gleichzeitig kann das strömende Spülfluid Kohlendioxid aus der Umgebung des Hohlkörpers 21, das durch die Wandung des Hohlkörpers 21 in den Innenraum diffundiert, aufnehmen, und aus dem medizinischen Platzhalter durch den Adapter 29 ableiten. Hierdurch kann die Umgebung des Hohlkörpers 21 mit Sauerstoff versorgt werden und durch die Aufnahme von Kohlendioxid eine Übersäuerung der Umgebung des Hohlkörpers 21 verhindert werden.

In dem Gaseinleitungsschlauch 22 und/oder in dem Gasausleitungsschlauch 23 können bevorzugt keimundurchlässige aber für Gase durchlässige Sterilfilter (nicht gezeigt) angeordnet sein. Das Spülfluid kann mit dem Sterilfilter von Keimen befreit werden, die ansonsten in den Hohlkörper 21 gelangen könnten und/oder die vom Hohlkörper 21 durch den Adapter 29 abgeleitet werden könnten. Hierdurch wird die Gefahr einer Infektion des behandelten Patienten und des behandelnden Personals reduziert.

Bevorzugt kann der Sterilfilter in dem Gaseinleitungsschlauch 22 oder dem Gasausleitungsschlauch 23 in Strömungsrichtung hinter dem Ventil 24 oder dem Ventil 25 angeordnet sein oder die Sterilfilter in dem Gaseinleitungsschlauch 22 und in dem Gasausleitungsschlauch 23 hinter den Ventilen 24, 25 angeordnet sein.

Es kann auch vorgesehen sein, dass der Hohlkörper 21 und/oder der Koaxialschlauch 38 mit einer antiseptischen Substanz beschichtet ist oder eine lösbare antiseptische Substanz in dem Material des Hohlkörpers 21 enthalten ist, um eine Infektion zu verhindern.

Zur Behandlung der Umgebung des Hohlkörpers 21 kann zudem vorgesehen sein, dass in zumindest einer Wandung des Hohlkörpers 21 oder des zumindest einen Kunststoffs des Hohlkörpers 21 Durchführungen (nicht gezeigt) angeordnet sind, die in Richtung der Außenseite des Hohlkörpers 21 weisende Öffnungen aufweisen, die zumindest einen Kanal (nicht gezeigt) mit der Umgebung des Hohlkörpers 21 für Flüssigkeiten und Gase durchlässig verbinden. Der zumindest eine Kanal kann zum Einleiten von Flüssigkeiten in den Hohlkörper 21 zu den Durchführungen vorgesehen sein. Ferner kann der zumindest eine Kanal mit einem Zuleitungsschlauch (nicht gezeigt) für pharmazeutische Wirkstofflösungen verbunden sein. Dadurch kann eine pharmazeutische Wirkstofflösung an der Oberfläche des Hohlkörpers 21 abgegeben werden.

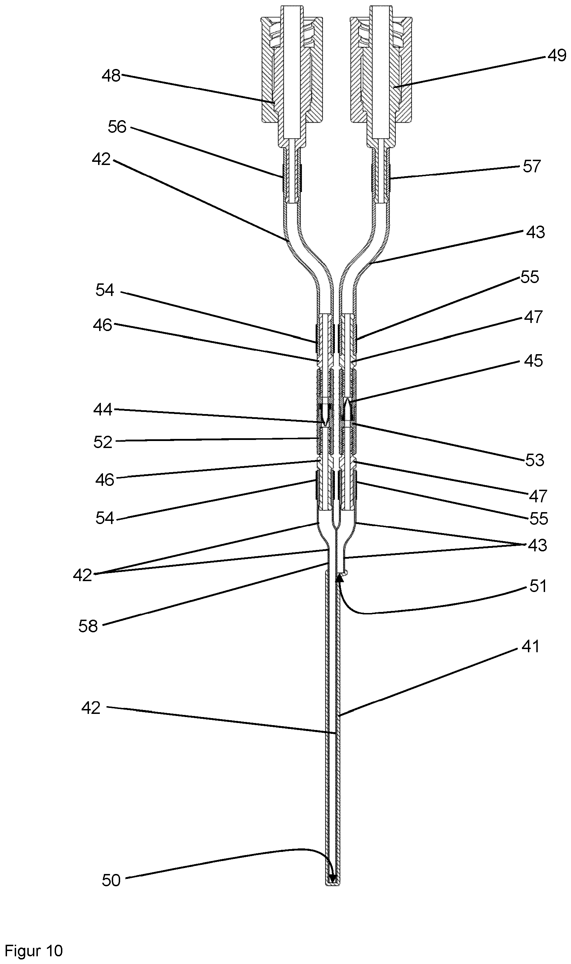

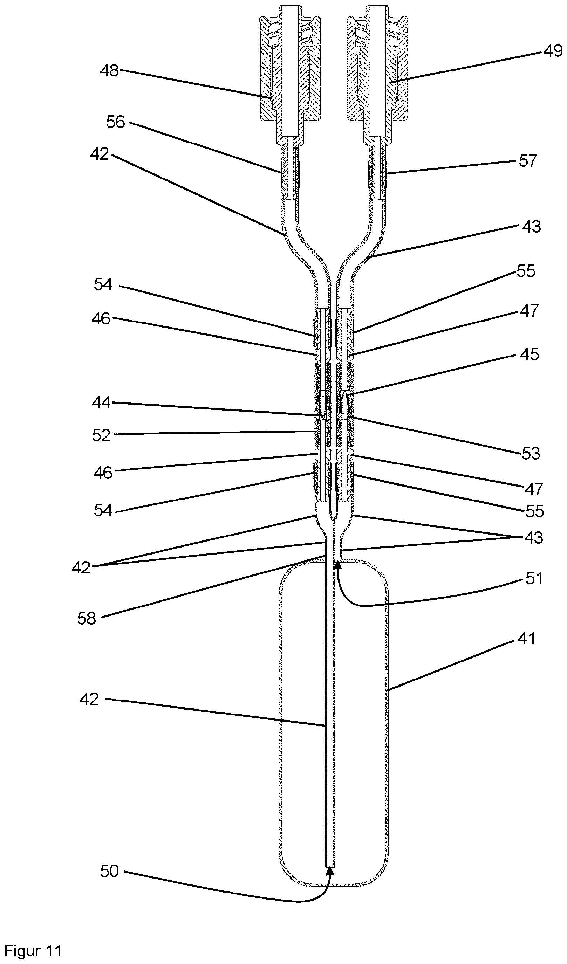

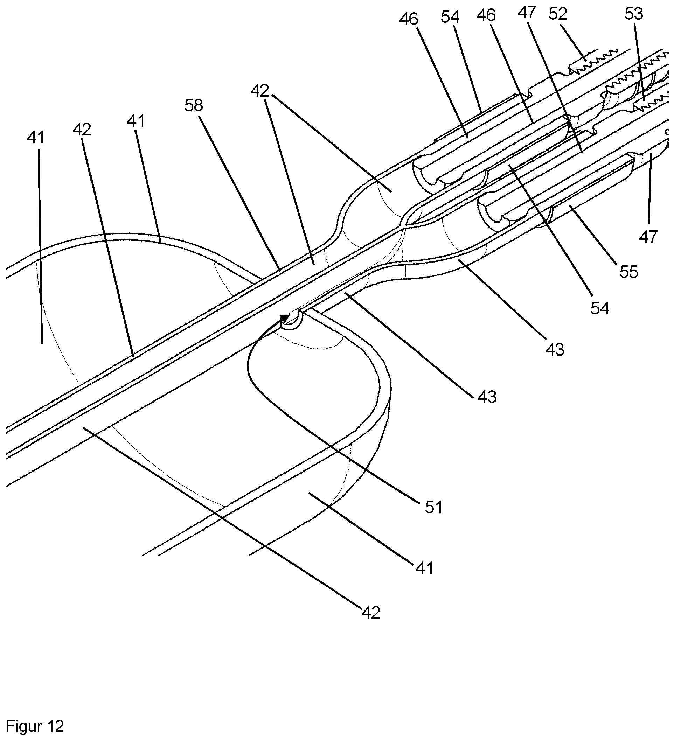

In den Figuren 10 bis 12 sind Abbildungen eines dritten erfindungsgemäßen medizinischen Platzhalters gezeigt. Die Figur 10 zeigt den Platzhalter im komprimierten Zustand und die Figuren 11 und 12 zeigen den Platzhalter im expandierten Zustand.

Der dritte erfindungsgemäße medizinische Platzhalter kann einen Hohlkörper 41 aus einem elastisch oder plastisch verformbaren Kunststoff aufweisen. Der Hohlkörper 41 kann expandierbar sein, indem er mit einem Spülgas aufgeblasen wird oder indem eine Spülflüssigkeit mit einem Druck in den Hohlkörper 41 eingedrückt wird. Der Hohlkörper 41 kann beispielsweise aus einem Gummi bestehen.

Das für den Hohlkörper 41 verwendete Material kann für gasförmigen molekularen Sauerstoff und für gasförmiges Kohlendioxid durchlässig sein. Der Hohlkörper 41 beziehungsweise das Material, aus dem der Hohlkörper 41 gefertigt ist, kann hierzu einen Permeabilitätskoeffizienten für Sauerstoff von größer oder gleich 0,5 cm³/(m²*d*bar) und einen Permeabilitätskoeffizienten für Kohlendioxid von größer oder gleich 0,5 cm³/(m²*d*bar) aufweisen. Der Permeabilitätskoeffizient wird nach DIN 53380-4 (11/2006) bestimmt.

Zum Einleiten eines Spülfluids (eines Spülgases oder einer Spülflüssigkeit) kann ein Innenraum des Hohlkörpers 41 mit einem Gaseinleitungsschlauch 42 aus Kunststoff verbunden sein. Zum Ableiten des Spülfluids (des Spülgases oder der Spülflüssigkeit) aus dem Hohlkörper 41 heraus kann der Innenraum des Hohlkörpers 41 mit einem Gasausleitungsschlauch 43 aus Kunststoff verbunden sein. Der Gaseinleitungsschlauch 42 und der Gasausleitungsschlauch 43 können zumindest bereichsweise flexibel und beweglich sein. In dem Gaseinleitungsschlauch 42 kann ein Ventil 44 in Form eines Lippenventils angeordnet sein, das eine Strömung des Spülfluids in Richtung des Hohlkörpers 41 ermöglicht aber eine Strömung des Spülfluids in der Richtung vom Hohlkörper 41 weg verhindert. In dem Gasausleitungsschlauch 43 kann ein Ventil 45 in Form eines Lippenventils angeordnet sein, das eine Strömung des Spülfluids in Richtung des Hohlkörpers 41 verhindert aber eine Strömung des Spülfluids in der Richtung vom Hohlkörper 41 weg erlaubt. Das Ventil 45 kann dazu ausgelegt sein, dass es ab einem Mindestdruck des Spülfluids öffnet. Bevorzugt ist der Mindestdruck am Ventil 45 einstellbar. Der Mindestdruck kann dabei so gewählt werden, dass der Druck des Spülfluids dazu ausreicht, den Hohlkörper 41 vom komprimierten Zustand (siehe Figur 10) in den expandierten Zustand zu überführen (siehe Figuren 11 und 12).

Die Ventile 44, 45 können über Verbindungshülsen 46, 47 mit dem Gaseinleitungsschlauch 42 und dem Gasausleitungsschlauch 43 verbunden sein. Hierzu kann der Gaseinleitungsschlauch 42 auf die Verbindungshülse 46 gesteckt sein und dort mit einer Krimphülse 54 befestigt sein. Ebenso kann der Gasausleitungsschlauch 43 auf die Verbindungshülse 47 gesteckt sein und dort mit einer Krimphülse 55 befestigt sein.

Der Gaseinleitungsschlauch 42 kann an seiner Rückseite in einem Adapter 48 in Form eines Luer-Lock-Adapters enden. Ebenso kann der Gasausleitungsschlauch 43 an seiner Rückseite in einem Adapter 49 in Form eines Luer-Lock-Adapters enden. Durch die Adapter 48, 49 kann das Spülfluid eingespeist und abgeleitet werden.

Der Gaseinleitungsschlauch 42 kann über eine Einströmöffnung 50 in den vorderen Teil des Innenraums des Hohlkörpers 41 münden, während der Gasausleitungsschlauch 43 über eine Ausströmöffnung 51 an der gegenüberliegenden Rückseite des Innenraums des Hohlkörpers 41 mit dem Innenraum des Hohlkörpers 41 verbunden sein kann. Hierdurch wird sichergestellt, dass das Spülfluid entlang der Oberfläche der Wandung des gesamten Hohlkörpers 41 strömen kann und dadurch ein Gasaustausch von Sauerstoff und Kohlendioxid durch die Wandung auf der gesamten Länge des Hohlkörpers 41 erfolgen kann.

Das Ventil 44 kann ein Ventilgehäuse 52 aufweisen. Das Ventilgehäuse 52 kann über ein Innengewinde mit Außengewinden der Verbindungshülsen 46 verbunden sein. Das Ventil 45 kann ein Ventilgehäuse 53 aufweisen. Das Ventilgehäuse 53 kann über Innengewinde mit Außengewinden der Verbindungshülsen 47 verbunden sein. Alle Verbindungen können gasdicht und Druckdicht ausgeführt sein.

Der Gaseinleitungsschlauch 42 kann auf einen Stutzen des Adapters 48 aufgesteckt sein und dort mit einer Krimphülse 56 druckdicht und gasdicht befestigt werden. Der Gasausleitungsschlauch 43 kann auf einen Stutzen des Adapters 49 aufgesteckt sein und dort mit einer Krimphülse 57 druckdicht und gasdicht befestigt werden.

Der Gaseinleitungsschlauch 42 und der Gasausleitungsschlauch 43 sind bei dem dritten Ausführungsbeispiel im Unterschied zum ersten Ausführungsbeispiel bereits vor dem Hohlkörper 41 zusammengeführt und bilden dort einen Doppelschlauch 58 mit zwei nebeneinander angeordneten Leitungen, die Teil des Gaseinleitungsschlauchs 42 und des Gasausleitungsschlauchs 43 sind. Dabei kann der Gasausleitungsschlauch 43 neben dem Gaseinleitungsschlauch 42 angeordnet sein (wie in den Figuren 10 bis 12 gezeigt). Der Vorteil ist, dass nur eine Verbindung vorhanden ist, so dass der Durchgang leichter zu schließen ist und die Gefahr für einen Durchtritt von Keimen reduziert ist. Der Gasausleitungsschlauch 43 und der Gaseinleitungsschlauch 42 haben im Bereich des Doppelschlauchs 58 einen halbmondförmigen beziehungsweise halbkreisförmigen Leitungsquerschnitt.

Im Betrieb kann das Spülfluid durch den Adapter 48 in den medizinischen Platzhalter eingespeist werden. Das Spülfluid kann durch den Gaseinleitungsschlauch 42 strömen und bei ausreichendem Druck das Ventil 44 öffnen. Dann kann das Spülfluid durch die Einströmöffnung 50 in den Hohlkörper 41 und durch den Hohlkörper 41 fließen. Das Spülfluid kann durch den Gasausleitungsschlauch 43 bis zum zunächst geschlossenen Ventil 45 strömen. Dabei kann sich im Inneren des Hohlkörpers 41 ein Druck aufbauen, mit dem der Hohlkörper 41 in den expandierten Zustand überführt wird. Sobald der Druck am Ventil 45 im Gasausleitungsschlauch 43 ausreicht, öffnet sich das Ventil 45 und das Spülfluid fließt durch den Gasausleitungsschlauch 43 und den Adapter 49 ab.

Der Hohlkörper 41 kann im komprimierten Zustand auch durch eine kleine Öffnung in einen Hohlraum eingebracht werden. Dort kann der Hohlkörper 41 expandiert werden und so die äußere Form des Hohlkörpers 41 an den Hohlraum angepasst werden. Der expandierte Hohlkörper 41, beziehungsweise der Platzhalter im expandierten Zustand, kann so den Hohlraum mechanisch stützen und stabilisieren. Wenn der Platzhalter nicht mehr benötigt wird, kann der Hohlkörper 41 wieder in den komprimierten Zustand überführt werden, beispielsweise indem er evakuiert wird. Der Hohlkörper 41 kann dann wieder durch eine enge Öffnung aus dem Hohlraum entfernt werden. Insbesondere wenn der Hohlraum eine Kavität in einem Knochen ist, kann dieser Knochendefekt so schonend behandelt werden.

In dem Spülfluid ist Sauerstoff enthalten. Das Spülfluid kann durch die Wandung des Hohlkörpers 41 Sauerstoff an die Umgebung des Hohlkörpers 41 abgeben. Gleichzeitig kann das strömende Spülfluid Kohlendioxid aus der Umgebung des Hohlkörpers 41, das durch die Wandung des Hohlkörpers 41 in den Innenraum diffundiert, aufnehmen, und aus dem medizinischen Platzhalter durch den Adapter 29 ableiten. Hierdurch kann die Umgebung des Hohlkörpers 41 mit Sauerstoff versorgt werden und durch die Aufnahme von Kohlendioxid eine Übersäuerung der Umgebung des Hohlkörpers 41 verhindert werden.

In dem Gaseinleitungsschlauch 42 und/oder in dem Gasausleitungsschlauch 43 können bevorzugt keimundurchlässige aber für Gase durchlässige Sterilfilter (nicht gezeigt) angeordnet sein. Das Spülfluid kann mit dem Sterilfilter von Keimen befreit werden, die ansonsten in den Hohlkörper 41 gelangen könnten und/oder die vom Hohlkörper 41 durch den Adapter 49 abgeleitet werden könnten. Hierdurch wird die Gefahr einer Infektion des behandelten Patienten und des behandelnden Personals reduziert.

Bevorzugt kann der Sterilfilter in dem Gaseinleitungsschlauch 42 oder dem Gasausleitungsschlauch 43 in Strömungsrichtung hinter dem Ventil 44 oder dem Ventil 45 angeordnet sein oder die Sterilfilter in dem Gaseinleitungsschlauch 42 und in dem Gasausleitungsschlauch 43 hinter den Ventilen 44, 45 angeordnet sein.

Es kann auch vorgesehen sein, dass der Hohlkörper 41 und/oder der Doppelschlauch 58 mit einer antiseptischen Substanz beschichtet ist oder eine lösbare antiseptische Substanz in dem Material des Hohlkörpers 41 enthalten ist, um eine Infektion zu verhindern.

Zur Behandlung der Umgebung des Hohlkörpers 41 kann zudem vorgesehen sein, dass in zumindest einer Wandung des Hohlkörpers 41 oder des zumindest einen Kunststoffs des Hohlkörpers 41 Durchführungen (nicht gezeigt) angeordnet sind, die in Richtung der Außenseite des Hohlkörpers 41 weisende Öffnungen aufweisen, die zumindest einen Kanal (nicht gezeigt) mit der Umgebung des Hohlkörpers 41 für Flüssigkeiten und Gase durchlässig verbinden. Der zumindest eine Kanal kann zum Einleiten von Flüssigkeiten in den Hohlkörper 41 zu den Durchführungen vorgesehen sein. Ferner kann der zumindest eine Kanal mit einem Zuleitungsschlauch (nicht gezeigt) für pharmazeutische Wirkstofflösungen verbunden sein. Dadurch kann eine pharmazeutische Wirkstofflösung an der Oberfläche des Hohlkörpers 41 abgegeben werden.

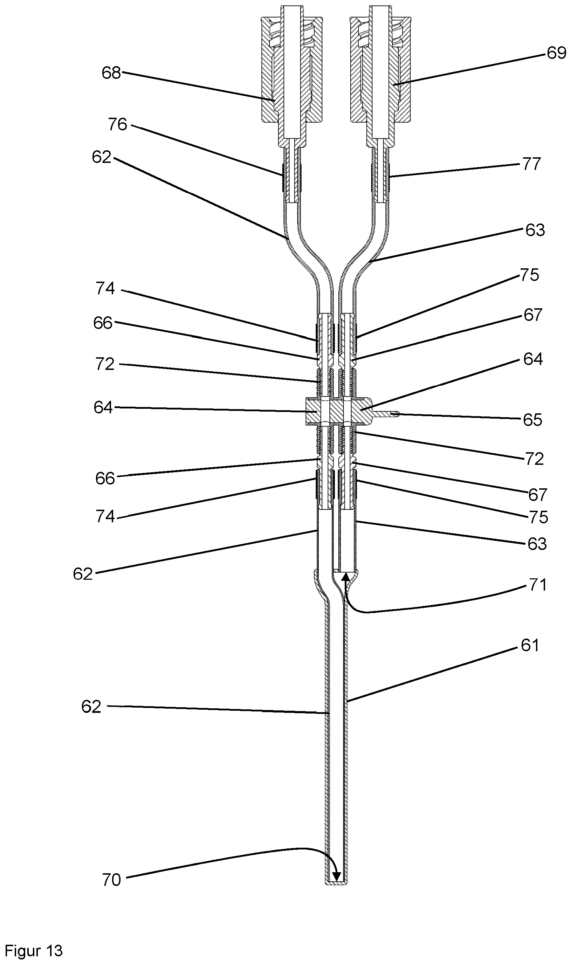

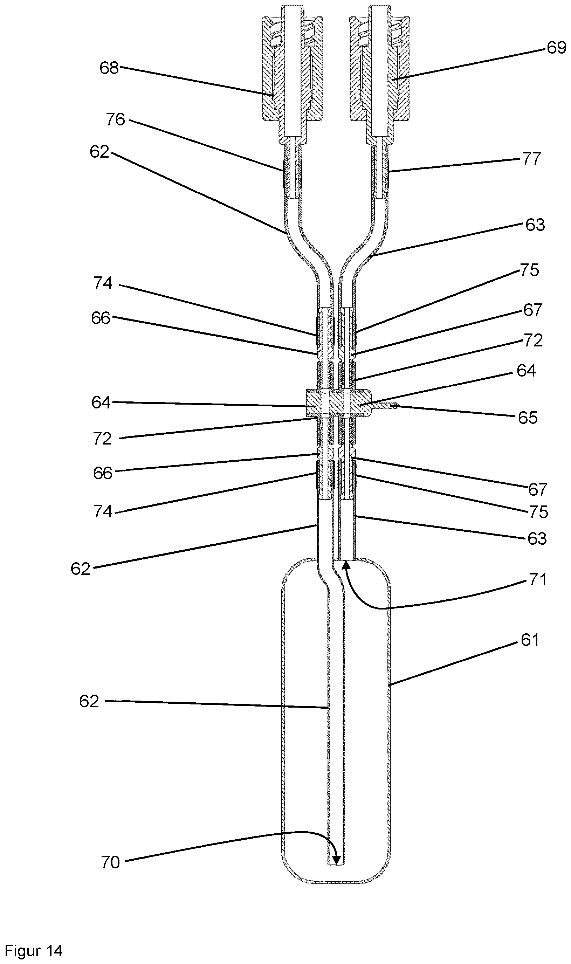

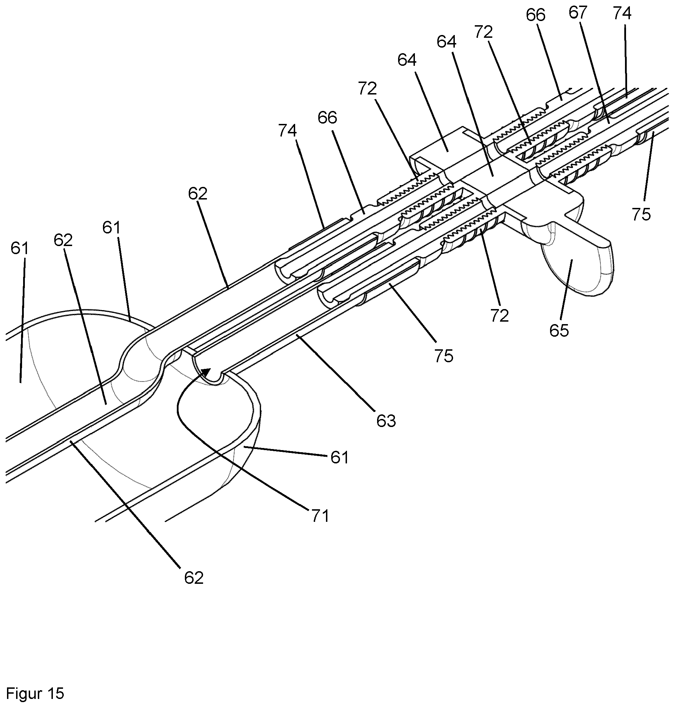

In den Figuren 13 bis 15 sind Abbildungen eines vierten erfindungsgemäßen medizinischen Platzhalters gezeigt. Die Figur 13 zeigt den Platzhalter im komprimierten Zustand und die Figuren 14 und 15 zeigen den Platzhalter im expandierten Zustand.

Der vierte erfindungsgemäße medizinische Platzhalter kann einen Hohlkörper 61 aus einem elastisch oder plastisch verformbaren Kunststoff aufweisen. Der Hohlkörper 61 kann expandierbar sein, indem er mit einem Spülgas aufgeblasen wird oder indem eine Spülflüssigkeit mit einem Druck in den Hohlkörper 61 eingedrückt wird. Der Hohlkörper 61 kann beispielsweise aus einem Gummi bestehen.

Das für den Hohlkörper 61 verwendete Material kann für gasförmigen molekularen Sauerstoff und für gasförmiges Kohlendioxid durchlässig sein. Der Hohlkörper 61 beziehungsweise das Material, aus dem der Hohlkörper 61 gefertigt ist, kann hierzu einen Permeabilitätskoeffizienten für Sauerstoff von größer oder gleich 0,5 cm³/(m²*d*bar) und einen Permeabilitätskoeffizienten für Kohlendioxid von größer oder gleich 0,5 cm³/(m²*d*bar) aufweisen. Der Permeabilitätskoeffizient wird nach DIN 53380-4 (11/2006) bestimmt.

Zum Einleiten eines Spülfluids (eines Spülgases oder einer Spülflüssigkeit) kann ein Innenraum des Hohlkörpers 61 mit einem Gaseinleitungsschlauch 62 aus Kunststoff verbunden sein. Zum Ableiten des Spülfluids (des Spülgases oder der Spülflüssigkeit) aus dem Hohlkörper 61 heraus kann der Innenraum des Hohlkörpers 61 mit einem Gasausleitungsschlauch 63 aus Kunststoff verbunden sein. Der Gaseinleitungsschlauch 62 und der Gasausleitungsschlauch 63 können zumindest bereichsweise flexibel und beweglich sein. In dem Gaseinleitungsschlauch 62 und in dem Gasausleitungsschlauch 63 kann ein Ventil 64 in Form eines manuell bedienbaren Drehventils angeordnet sein, das über einen Ventilgriff 65 bedienbar ist. Mit dem Ventil 64 kann der Gaseinleitungsschlauch 62 und der Gasausleitungsschlauch 63 manuell geschlossen und geöffnet werden. Es kann auch vorgesehen sein, dass der Gaseinleitungsschlauch 62 und der Gasausleitungsschlauch 63 einzeln und unabhängig voneinander manuell geschlossen und geöffnet werden können. Ein durch den Querschnitt des Gasausleitungsschlauchs 63 bestimmter Staudruck kann dabei so gewählt werden, dass der Druck des Spülfluids dazu ausreicht, den Hohlkörper 61 bei geöffnetem Ventil 64 vom komprimierten Zustand (siehe Figur 13) in den expandierten Zustand zu überführen (siehe Figuren 14 und 15).

Das Ventil 64 kann über Verbindungshülsen 66, 67 mit dem Gaseinleitungsschlauch 62 und dem Gasausleitungsschlauch 63 verbunden sein. Hierzu kann der Gaseinleitungsschlauch 62 auf die Verbindungshülse 66 gesteckt sein und dort mit einer Krimphülse 74 befestigt sein. Ebenso kann der Gasausleitungsschlauch 63 auf die Verbindungshülse 67 gesteckt sein und dort mit einer Krimphülse 75 befestigt sein.

Der Gaseinleitungsschlauch 62 kann an seiner Rückseite in einem Adapter 68 in Form eines Luer-Lock-Adapters enden. Ebenso kann der Gasausleitungsschlauch 63 an seiner Rückseite in einem Adapter 69 in Form eines Luer-Lock-Adapters enden. Durch die Adapter 68, 69 kann das Spülfluid eingespeist und abgeleitet werden.

Der Gaseinleitungsschlauch 62 kann über eine Einströmöffnung 70 in den vorderen Teil des Innenraums des Hohlkörpers 61 münden, während der Gasausleitungsschlauch 63 über eine Ausströmöffnung 71 an der gegenüberliegenden Rückseite des Innenraums des Hohlkörpers 61 mit dem Innenraum des Hohlkörpers 61 verbunden sein kann. Hierdurch wird sichergestellt, dass das Spülfluid entlang der Oberfläche der Wandung des gesamten Hohlkörpers 61 strömen kann und dadurch ein Gasaustausch von Sauerstoff und Kohlendioxid durch die Wandung auf der gesamten Länge des Hohlkörpers 61 erfolgen kann.

Das Ventil 64 kann ein Ventilgehäuse 72 aufweisen. Das Ventilgehäuse 72 kann über ein Innengewinde mit Außengewinden der Verbindungshülsen 66 und der Verbindungshülsen 67 verbunden sein. Alle Verbindungen können gasdicht und Druckdicht ausgeführt sein.

Der Gaseinleitungsschlauch 62 kann auf einen Stutzen des Adapters 68 aufgesteckt sein und dort mit einer Krimphülse 76 druckdicht und gasdicht befestigt werden. Der Gasausleitungsschlauch 63 kann auf einen Stutzen des Adapters 69 aufgesteckt sein und dort mit einer Krimphülse 77 druckdicht und gasdicht befestigt werden.

Im Betrieb kann das Spülfluid durch den Adapter 68 in den medizinischen Platzhalter eingespeist werden. Das Spülfluid kann bei geöffnetem Ventil 64 durch den Gaseinleitungsschlauch 62 strömen. Dann kann das Spülfluid durch die Einströmöffnung 70 in den Hohlkörper 61 und durch den Hohlkörper 61 fließen. Dabei kann sich im Inneren des Hohlkörpers 61 ein Druck aufbauen, mit dem der Hohlkörper 61 in den expandierten Zustand überführt wird.

Der Hohlkörper 61 kann im komprimierten Zustand bei geschlossenem Ventil 64 auch durch eine kleine Öffnung in einen Hohlraum eingebracht werden. Dort kann der Hohlkörper 61 expandiert werden und so die äußere Form des Hohlkörpers 61 an den Hohlraum angepasst werden. Der expandierte Hohlkörper 61, beziehungsweise der Platzhalter im expandierten Zustand, kann so den Hohlraum mechanisch stützen und stabilisieren. Wenn der Platzhalter nicht mehr benötigt wird, kann der Hohlkörper 61 wieder in den komprimierten Zustand überführt werden, beispielsweise indem er evakuiert wird. Der Hohlkörper 61 kann dann wieder durch eine enge Öffnung aus dem Hohlraum entfernt werden. Insbesondere wenn der Hohlraum eine Kavität in einem Knochen ist, kann dieser Knochendefekt so schonend behandelt werden.

In dem Spülfluid ist Sauerstoff enthalten. Das Spülfluid kann durch die Wandung des Hohlkörpers 61 Sauerstoff an die Umgebung des Hohlkörpers 61 abgeben. Gleichzeitig kann das strömende Spülfluid Kohlendioxid aus der Umgebung des Hohlkörpers 61, das durch die Wandung des Hohlkörpers 61 in den Innenraum diffundiert, aufnehmen, und aus dem medizinischen Platzhalter durch den Adapter 69 ableiten. Hierdurch kann die Umgebung des Hohlkörpers 61 mit Sauerstoff versorgt werden und durch die Aufnahme von Kohlendioxid eine Übersäuerung der Umgebung des Hohlkörpers 61 verhindert werden.

In dem Gaseinleitungsschlauch 62 und/oder in dem Gasausleitungsschlauch 63 können bevorzugt keimundurchlässige aber für Gase durchlässige Sterilfilter (nicht gezeigt) angeordnet sein. Das Spülfluid kann mit dem Sterilfilter von Keimen befreit werden, die ansonsten in den Hohlkörper 61 gelangen könnten und/oder die vom Hohlkörper 61 durch den Adapter 69 abgeleitet werden könnten. Hierdurch wird die Gefahr einer Infektion des behandelten Patienten und des behandelnden Personals reduziert.

Bevorzugt kann der Sterilfilter in dem Gaseinleitungsschlauch 62 oder dem Gasausleitungsschlauch 63 in Strömungsrichtung hinter dem Ventil 64 angeordnet sein oder die Sterilfilter in dem Gaseinleitungsschlauch 62 und in dem Gasausleitungsschlauch 63 hinter den Ventilen 64 angeordnet sein.

Es kann auch vorgesehen sein, dass der Hohlkörper 61 und/oder die Schläuche 62, 63 im Bereich des Hohlkörpers 61 mit einer antiseptischen Substanz beschichtet ist oder eine lösbare antiseptische Substanz in dem Material des Hohlkörpers 61 enthalten ist, um eine Infektion zu verhindern.

Zur Behandlung der Umgebung des Hohlkörpers 61 kann zudem vorgesehen sein, dass in zumindest einer Wandung des Hohlkörpers 61 oder des zumindest einen Kunststoffs des Hohlkörpers 61 Durchführungen (nicht gezeigt) angeordnet sind, die in Richtung der Außenseite des Hohlkörpers 61 weisende Öffnungen aufweisen, die zumindest einen Kanal (nicht gezeigt) mit der Umgebung des Hohlkörpers 61 für Flüssigkeiten und Gase durchlässig verbinden. Der zumindest eine Kanal kann zum Einleiten von Flüssigkeiten in den Hohlkörper 61 zu den Durchführungen vorgesehen sein. Ferner kann der zumindest eine Kanal mit einem Zuleitungsschlauch (nicht gezeigt) für pharmazeutische Wirkstofflösungen verbunden sein. Dadurch kann eine pharmazeutische Wirkstofflösung an der Oberfläche des Hohlkörpers 61 abgegeben werden.

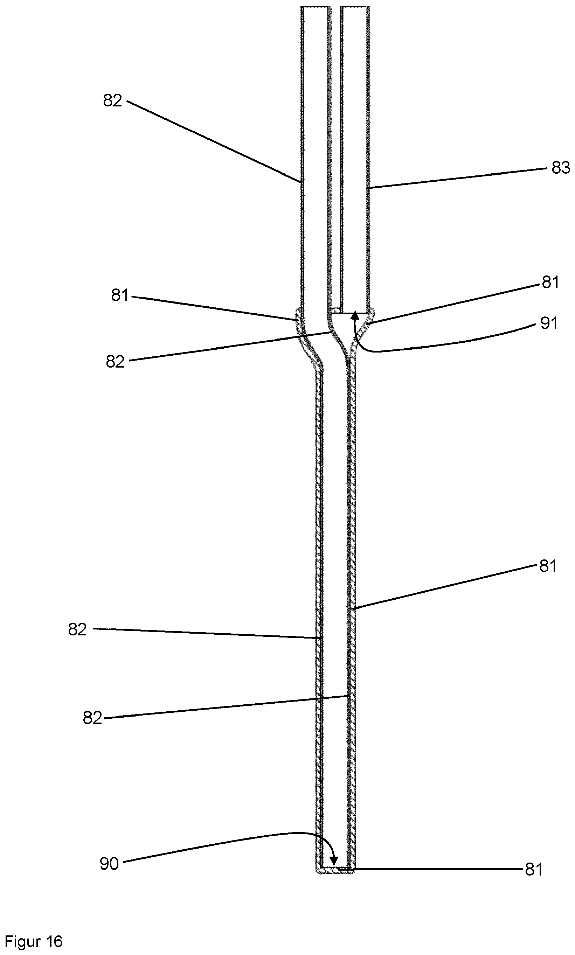

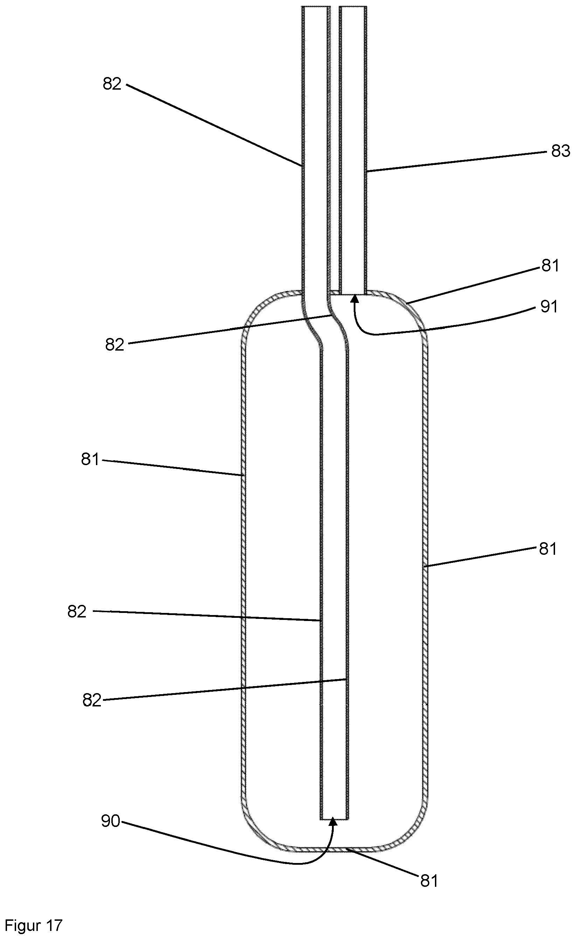

In den Figuren 16 und 17 sind Abbildungen eines sehr einfach aufgebauten fünften erfindungsgemäßen medizinischen Platzhalters gezeigt. Die Figur 16 zeigt den Platzhalter im komprimierten Zustand und die Figur 17 zeigt den Platzhalter im expandierten Zustand.

Der fünfte erfindungsgemäße medizinische Platzhalter kann einen Hohlkörper 81 aus einem elastisch oder plastisch verformbaren Kunststoff aufweisen. Der Hohlkörper 81 kann expandierbar sein, indem er mit einem Spülgas aufgeblasen wird oder indem eine Spülflüssigkeit mit einem Druck in den Hohlkörper 81 eingedrückt wird. Der Hohlkörper 81 kann beispielsweise aus einem Gummi bestehen.

Das für den Hohlkörper 81 verwendete Material kann für gasförmigen molekularen Sauerstoff und für gasförmiges Kohlendioxid durchlässig sein. Der Hohlkörper 81 beziehungsweise das Material, aus dem der Hohlkörper 81 gefertigt ist, kann hierzu einen Permeabilitätskoeffizienten für Sauerstoff von größer oder gleich 0,5 cm³/(m²*d*bar) und einen Permeabilitätskoeffizienten für Kohlendioxid von größer oder gleich 0,5 cm³/(m²*d*bar) aufweisen. Der Permeabilitätskoeffizient wird nach DIN 53380-4 (11/2006) bestimmt.

Zum Einleiten eines Spülfluids (eines Spülgases oder einer Spülflüssigkeit) kann ein Innenraum des Hohlkörpers 81 mit einem Gaseinleitungsschlauch 82 aus Kunststoff verbunden sein. Zum Ableiten des Spülfluids (des Spülgases oder der Spülflüssigkeit) aus dem Hohlkörper 81 heraus kann der Innenraum des Hohlkörpers 81 mit einem Gasausleitungsschlauch 83 aus Kunststoff verbunden sein. Der Gaseinleitungsschlauch 82 und der Gasausleitungsschlauch 83 können zumindest bereichsweise flexibel und beweglich sein. Ein durch den Querschnitt des Gasausleitungsschlauchs 83 bestimmter Staudruck kann dabei so gewählt werden, dass der Druck des Spülfluids dazu ausreicht, den Hohlkörper 81 vom komprimierten Zustand (siehe Figur 16) in den expandierten Zustand zu überführen (siehe Figur 17).

Der Gaseinleitungsschlauch 82 kann über eine Einströmöffnung 90 in den vorderen Teil des Innenraums des Hohlkörpers 81 münden, während der Gasausleitungsschlauch 83 über eine Ausströmöffnung 91 an der gegenüberliegenden Rückseite des Innenraums des Hohlkörpers 81 mit dem Innenraum des Hohlkörpers 81 verbunden sein kann. Hierdurch wird sichergestellt, dass das Spülfluid entlang der Oberfläche der Wandung des gesamten Hohlkörpers 81 strömen kann und dadurch ein Gasaustausch von Sauerstoff und Kohlendioxid durch die Wandung auf der gesamten Länge des Hohlkörpers 81 erfolgen kann.

Im Betrieb kann das Spülfluid durch den Gaseinleitungsschlauch 82 in den medizinischen Platzhalter eingespeist werden. Das Spülfluid kann durch die Einströmöffnung 90 in den Hohlkörper 81 und durch den Hohlkörper 81 fließen. Dabei kann sich im Inneren des Hohlkörpers 81 ein Druck aufbauen, mit dem der Hohlkörper 81 in den expandierten Zustand überführt wird.

Der Hohlkörper 81 kann im komprimierten Zustand auch durch eine kleine Öffnung in einen Hohlraum eingebracht werden. Dort kann der Hohlkörper 81 expandiert werden und so die äußere Form des Hohlkörpers 81 an den Hohlraum angepasst werden. Der expandierte Hohlkörper 81, beziehungsweise der Platzhalter im expandierten Zustand, kann so den Hohlraum mechanisch stützen und stabilisieren. Wenn der Platzhalter nicht mehr benötigt wird, kann der Hohlkörper 81 wieder in den komprimierten Zustand überführt werden, beispielsweise indem er evakuiert wird. Der Hohlkörper 81 kann dann wieder durch eine enge Öffnung aus dem Hohlraum entfernt werden. Insbesondere wenn der Hohlraum eine Kavität in einem Knochen ist, kann dieser Knochendefekt so schonend behandelt werden.

In dem Spülfluid ist Sauerstoff enthalten. Das Spülfluid kann durch die Wandung des Hohlkörpers 81 Sauerstoff an die Umgebung des Hohlkörpers 81 abgeben. Gleichzeitig kann das strömende Spülfluid Kohlendioxid aus der Umgebung des Hohlkörpers 81, das durch die Wandung des Hohlkörpers 81 in den Innenraum diffundiert, aufnehmen, und aus dem medizinischen Platzhalter durch den Gasausleitungsschlauch 83 ableiten. Hierdurch kann die Umgebung des Hohlkörpers 81 mit Sauerstoff versorgt werden und durch die Aufnahme von Kohlendioxid eine Übersäuerung der Umgebung des Hohlkörpers 81 verhindert werden.

In dem Gaseinleitungsschlauch 82 und/oder in dem Gasausleitungsschlauch 83 können bevorzugt keimundurchlässige aber für Gase durchlässige Sterilfilter (nicht gezeigt) angeordnet sein. Das Spülfluid kann mit dem Sterilfilter von Keimen befreit werden, die ansonsten in den Hohlkörper 81 gelangen könnten und/oder die vom Hohlkörper 81 durch den Gasausleitungsschlauch 83 abgeleitet werden könnten. Hierdurch wird die Gefahr einer Infektion des behandelten Patienten und des behandelnden Personals reduziert.

Es kann auch vorgesehen sein, dass der Hohlkörper 81 und/oder die Schläuche 82, 83 im Bereich des Hohlkörpers 81 mit einer antiseptischen Substanz beschichtet ist oder eine lösbare antiseptische Substanz in dem Material des Hohlkörpers 81 enthalten ist, um eine Infektion zu verhindern.

Zur Behandlung der Umgebung des Hohlkörpers 81 kann zudem vorgesehen sein, dass in zumindest einer Wandung des Hohlkörpers 81 oder des zumindest einen Kunststoffs des Hohlkörpers 81 Durchführungen (nicht gezeigt) angeordnet sind, die in Richtung der Außenseite des Hohlkörpers 81 weisende Öffnungen aufweisen, die zumindest einen Kanal (nicht gezeigt) mit der Umgebung des Hohlkörpers 81 für Flüssigkeiten und Gase durchlässig verbinden. Der zumindest eine Kanal kann zum Einleiten von Flüssigkeiten in den Hohlkörper 81 zu den Durchführungen vorgesehen sein. Ferner kann der zumindest eine Kanal mit einem Zuleitungsschlauch (nicht gezeigt) für pharmazeutische Wirkstofflösungen verbunden sein. Dadurch kann eine pharmazeutische Wirkstofflösung an der Oberfläche des Hohlkörpers 81 abgegeben werden.

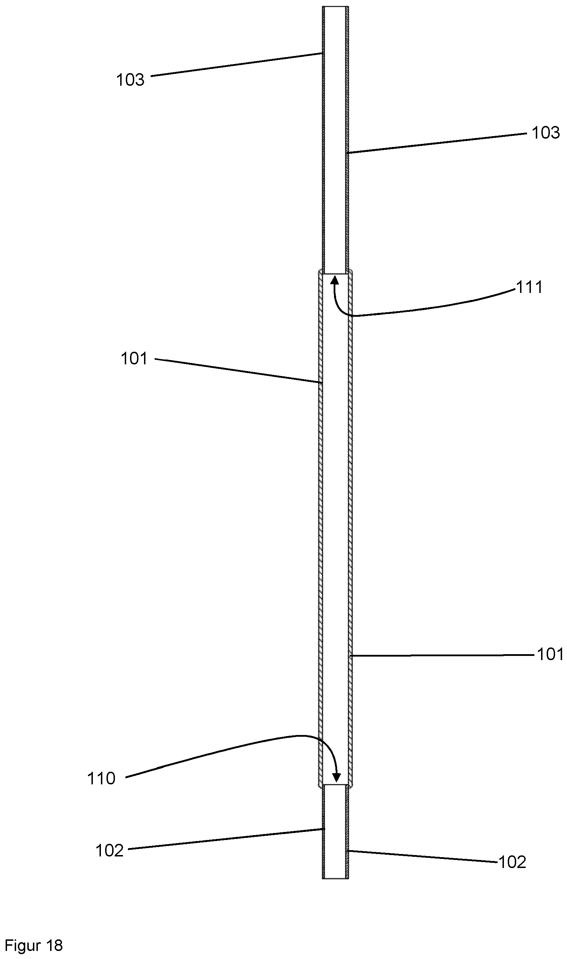

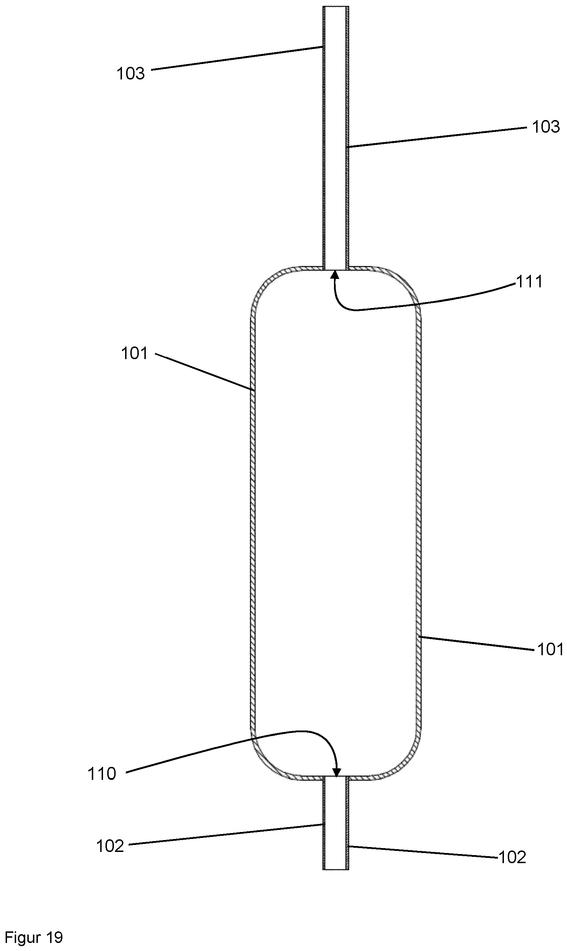

In den Figuren 18 und 19 sind Abbildungen eines sehr einfach aufgebauten sechsten erfindungsgemäßen medizinischen Platzhalters gezeigt. Die Figur 18 zeigt den Platzhalter im komprimierten Zustand und die Figur 19 zeigt den Platzhalter im expandierten Zustand.

Der sechste erfindungsgemäße medizinische Platzhalter kann einen Hohlkörper 101 aus einem elastisch oder plastisch verformbaren Kunststoff aufweisen. Der Hohlkörper 101 kann expandierbar sein, indem er mit einem Spülgas aufgeblasen wird oder indem eine Spülflüssigkeit mit einem Druck in den Hohlkörper 101 eingedrückt wird. Der Hohlkörper 81 kann beispielsweise aus einem Gummi bestehen.

Das für den Hohlkörper 101 verwendete Material kann für gasförmigen molekularen Sauerstoff und für gasförmiges Kohlendioxid durchlässig sein. Der Hohlkörper 101 beziehungsweise das Material, aus dem der Hohlkörper 101 gefertigt ist, kann hierzu einen Permeabilitätskoeffizienten für Sauerstoff von größer oder gleich 0,5 cm³/(m²*d*bar) und einen Permeabilitätskoeffizienten für Kohlendioxid von größer oder gleich 0,5 cm³/(m²*d*bar) aufweisen. Der Permeabilitätskoeffizient wird nach DIN 53380-4 (11/2006) bestimmt.

Zum Einleiten eines Spülfluids (eines Spülgases oder einer Spülflüssigkeit) kann ein Innenraum des Hohlkörpers 101 mit einem Gaseinleitungsschlauch 102 aus Kunststoff verbunden sein. Zum Ableiten des Spülfluids (des Spülgases oder der Spülflüssigkeit) aus dem Hohlkörper 101 heraus kann der Innenraum des Hohlkörpers 101 mit einem Gasausleitungsschlauch 103 aus Kunststoff verbunden sein. Der Gaseinleitungsschlauch 102 und der Gasausleitungsschlauch 103 können zumindest bereichsweise flexibel und beweglich sein. Ein durch den Querschnitt des Gasausleitungsschlauchs 103 bestimmter Staudruck kann dabei so gewählt werden, dass der Druck des Spülfluids dazu ausreicht, den Hohlkörper 101 vom komprimierten Zustand (siehe Figur 18) in den expandierten Zustand zu überführen (siehe Figur 19).

Der Gaseinleitungsschlauch 102 kann über eine Einströmöffnung 110 in den vorderen Teil des Innenraums des Hohlkörpers 101 münden, während der Gasausleitungsschlauch 103 über eine Ausströmöffnung 111 an der gegenüberliegenden Rückseite des Innenraums des Hohlkörpers 101 mit dem Innenraum des Hohlkörpers 101 verbunden sein kann. Hierdurch wird sichergestellt, dass das Spülfluid entlang der Oberfläche der Wandung des gesamten Hohlkörpers 101 strömen kann und dadurch ein Gasaustausch von Sauerstoff und Kohlendioxid durch die Wandung auf der gesamten Länge des Hohlkörpers 101 erfolgen kann.

Im Betrieb kann das Spülfluid durch den Gaseinleitungsschlauch 102 in den medizinischen Platzhalter eingespeist werden. Das Spülfluid kann durch die Einströmöffnung 110 in den Hohlkörper 101 und durch den Hohlkörper 101 fließen. Dabei kann sich im Inneren des Hohlkörpers 101 ein Druck aufbauen, mit dem der Hohlkörper 101 in den expandierten Zustand überführt wird.

Der Hohlkörper 101 kann im komprimierten Zustand auch durch eine kleine Öffnung in einen Hohlraum eingebracht werden. Dort kann der Hohlkörper 101 expandiert werden und so die äußere Form des Hohlkörpers 101 an den Hohlraum angepasst werden. Der expandierte Hohlkörper 101, beziehungsweise der Platzhalter im expandierten Zustand, kann so den Hohlraum mechanisch stützen und stabilisieren. Wenn der Platzhalter nicht mehr benötigt wird, kann der Hohlkörper 101 wieder in den komprimierten Zustand überführt werden, beispielsweise indem er evakuiert wird. Der Hohlkörper 101 kann dann wieder durch eine enge Öffnung aus dem Hohlraum entfernt werden. Insbesondere wenn der Hohlraum eine Kavität in einem Knochen ist, kann dieser Knochendefekt so schonend behandelt werden.

In dem Spülfluid ist Sauerstoff enthalten. Das Spülfluid kann durch die Wandung des Hohlkörpers 101 Sauerstoff an die Umgebung des Hohlkörpers 101 abgeben. Gleichzeitig kann das strömende Spülfluid Kohlendioxid aus der Umgebung des Hohlkörpers 101, das durch die Wandung des Hohlkörpers 101 in den Innenraum diffundiert, aufnehmen, und aus dem medizinischen Platzhalter durch den Gasausleitungsschlauch 103 ableiten. Hierdurch kann die Umgebung des Hohlkörpers 101 mit Sauerstoff versorgt werden und durch die Aufnahme von Kohlendioxid eine Übersäuerung der Umgebung des Hohlkörpers 101 verhindert werden.

In dem Gaseinleitungsschlauch 102 und/oder in dem Gasausleitungsschlauch 103 können bevorzugt keimundurchlässige aber für Gase durchlässige Sterilfilter (nicht gezeigt) angeordnet sein. Das Spülfluid kann mit dem Sterilfilter von Keimen befreit werden, die ansonsten in den Hohlkörper 101 gelangen könnten und/oder die vom Hohlkörper 101 durch den Gasausleitungsschlauch 103 abgeleitet werden könnten. Hierdurch wird die Gefahr einer Infektion des behandelten Patienten und des behandelnden Personals reduziert.

Es kann auch vorgesehen sein, dass der Hohlkörper 101 und/oder die Schläuche 102, 103 im Bereich des Hohlkörpers 101 mit einer antiseptischen Substanz beschichtet ist oder eine lösbare antiseptische Substanz in dem Material des Hohlkörpers 101 enthalten ist, um eine Infektion zu verhindern.

Zur Behandlung der Umgebung des Hohlkörpers 101 kann zudem vorgesehen sein, dass in zumindest einer Wandung des Hohlkörpers 101 oder des zumindest einen Kunststoffs des Hohlkörpers 101 Durchführungen (nicht gezeigt) angeordnet sind, die in Richtung der Außenseite des Hohlkörpers 101 weisende Öffnungen aufweisen, die zumindest einen Kanal (nicht gezeigt) mit der Umgebung des Hohlkörpers 101 für Flüssigkeiten und Gase durchlässig verbinden. Der zumindest eine Kanal kann zum Einleiten von Flüssigkeiten in den Hohlkörper 101 zu den Durchführungen vorgesehen sein. Ferner kann der zumindest eine Kanal mit einem Zuleitungsschlauch (nicht gezeigt) für pharmazeutische Wirkstofflösungen verbunden sein. Dadurch kann eine pharmazeutische Wirkstofflösung an der Oberfläche des Hohlkörpers 101 abgegeben werden.

### Bezugszeichenliste

- 1, 21, 41, 61, 81, 101: Hohlkörper
- 2, 22, 42, 62, 82, 102: Gaseinleitungsschlauch
- 3, 23, 43, 63, 83, 103: Gasausleitungsschlauch
- 4, 5, 24, 25, 44, 45: Ventil / Lippenventil / Einwegventil
- 6, 26, 46, 66: Verbindungshülse
- 7, 27, 47, 67: Verbindungshülse
- 8, 28, 48, 68: Adapter
- 9, 29, 49, 69: Adapter
- 10, 30, 50, 70, 90, 110: Einströmöffnung
- 11, 31, 51, 71, 91, 111: Ausströmöffnung
- 12, 32, 52, 72: Ventilgehäuse
- 13, 33, 53: Ventilgehäuse
- 14, 34, 54, 74: Krimphülse
- 15, 35, 55, 75: Krimphülse
- 16, 36, 56, 76: Krimphülse
- 17, 37, 57, 77: Krimphülse
- 38: Koaxialschlauch
- 58: Doppelschlauch
- 64: Ventil
- 65: Ventilgriff

## Patentansprüche

1. Medizinischer Platzhalter zur temporären Implantation in Knochenkavitäten, aufweisend mindestens einen Hohlkörper (1, 21, 41, 61, 81, 101), der expandierbar ist und der einen Innenraum im Inneren des Hohlkörpers (1, 21, 41, 61, 81, 101) begrenzt, einen Gaseinleitungsschlauch (2, 22, 42, 62, 82, 102), der mit dem Innenraum des Hohlkörpers (1, 21, 41, 61, 81, 101) gasdurchlässig verbunden oder verbindbar ist, einen Gasausleitungsschlauch (3, 23, 43, 63, 83, 103), der mit dem Innenraum des Hohlkörpers (1, 21, 41, 61, 81, 101) gasdurchlässig verbunden oder verbindbar ist, wobei der Hohlkörper (1, 21, 41, 61, 81, 101) zumindest bereichsweise oder vollständig aus zumindest einem Kunststoff besteht, wobei der Hohlkörper (1, 21, 41, 61, 81, 101) oder der zumindest eine Kunststoff des Hohlkörpers (1, 21, 41, 61, 81, 101) einen Permeabilitätskoeffizienten für Sauerstoff von größer oder gleich 0,5 cm³/(m²*d*bar) und einen Permeabilitätskoeffizienten für Kohlendioxid von größer oder gleich 0,5 cm³/(m²*d*bar) aufweist, und
wobei in dem Gaseinleitungsschlauch (2, 22, 42, 62, 82, 102) ein Einwegventil angeordnet ist und in dem Gasausleitungsschlauch (3, 23, 43, 63, 83, 103) ein Überdruckventil angeordnet ist.

2. Medizinischer Platzhalter nach Anspruch 1 wobei der Hohlkörper (1, 21, 41, 61, 81, 101) oder der zumindest eine Kunststoff des Hohlkörpers (1, 21, 41, 61, 81, 101) einen Permeabilitätskoeffizienten für Sauerstoff von größer oder gleich 1 cm³/(m²*d*bar) und einen Permeabilitätskoeffizienten für Kohlendioxid von größer oder gleich 1 cm³/(m²*d*bar) aufweist.

3. Medizinischer Platzhalter nach Anspruch 1 oder 2, wobei eine Einströmöffnung (10, 30, 50, 70, 90, 110) des Gaseinleitungsschlauchs (2, 22, 42, 62, 82, 102), mit der der Gaseinleitungsschlauch (2, 22, 42, 62, 82, 102) in den Innenraum mündet, räumlich getrennt von einer Ausströmöffnung (11, 31, 51, 71, 91, 111) des Gasausleitungsschlauchs (3, 23, 43, 63, 83, 103) angeordnet ist, wobei die Ausströmöffnung (11, 31, 51, 71, 91, 111) die Einmündung des Innenraums in den Gasausleitungsschlauch (3, 23, 43, 63, 83, 103) bildet.

4. Medizinischer Platzhalter nach Anspruch 3, wobei die Einströmöffnung (10, 30, 50, 70, 90, 110) des Gaseinleitungsschlauchs (2, 22, 42, 62, 82, 102) an einem ersten Ende des Hohlkörpers (1, 21, 41, 61, 81, 101) angeordnet ist und die Ausströmöffnung (11, 31, 51, 71, 91, 111) an einem dem ersten Ende gegenüberliegenden zweiten Ende des Hohlkörpers (1, 21, 41, 61, 81, 101) angeordnet ist.

5. Medizinischer Platzhalter nach einem der vorangehenden Ansprüche, wobei der Gaseinleitungsschlauch (22) und der Gasausleitungsschlauch (23) bereichsweise koaxial zueinander angeordnet sind, wobei vorzugsweise beide gemeinsam durch eine Durchführung aus dem Hohlkörper (21) geführt sind.

6. Medizinischer Platzhalter nach einem der vorangehenden Ansprüche, wobei der Gaseinleitungsschlauch (2, 22, 42, 62, 82, 102) und der Gasausleitungsschlauch (3, 23, 43, 63, 83, 103) miteinander verbunden sind.

7. Medizinischer Platzhalter nach einem der vorangehenden Ansprüche, wobei der Gaseinleitungsschlauch (22, 42) und der Gasausleitungsschlauch (23, 43) einen gemeinsamen Außenschlauch (38, 58) aufweisen, wobei besonders bevorzugt der gemeinsame Außenschlauch (38, 58) durch eine Trennwand unterteilt ist, die den Gaseinleitungsschlauch (22, 42) auf einer Seite begrenzt und den Gasausleitungsschlauch (23, 43) auf der anderen Seite begrenzt.

8. Medizinischer Platzhalter nach einem der vorangehenden Ansprüche, wobei in dem Gaseinleitungsschlauch (2, 22, 42, 62, 82, 102) und/oder dem Gasausleitungsschlauch (3, 23, 43, 63, 83, 103) ein keimundurchlässiger aber für Gase durchlässiger Sterilfilter angeordnet ist.

9. Medizinischer Platzhalter nach Anspruch 8, wobei der Sterilfilter in dem Gaseinleitungsschlauch (2, 22, 42, 62, 82, 102) oder dem Gasausleitungsschlauch (3, 23, 43, 63, 83, 103) in Strömungsrichtung hinter dem Einwegventil angeordnet ist oder die Sterilfilter in dem Gaseinleitungsschlauch (2, 22, 42, 62, 82, 102) und in dem Gasausleitungsschlauch (3, 23, 43, 63, 83, 103) hinter dem Einwegventil und hinter dem Überdruckventil angeordnet sind.

10. Medizinischer Platzhalter nach einem der vorangehenden Ansprüche, wobei der Öffnungsdruck des Überdruckventils einstellbar ist, und/oder der Gaseinleitungsschlauch (2, 22, 42, 62, 82, 102) und der Gasausleitungsschlauch (3, 23, 43, 63, 83, 103) aus einem für Sauerstoff und Kohlendioxid nicht durchlässigen Material bestehen, vorzugsweise aus einem für Sauerstoff und Kohlendioxid nicht durchlässigen Kunststoff bestehen.

11. Medizinischer Platzhalter nach einem der vorangehenden Ansprüche, wobei der Hohlkörper (1, 21, 41, 61, 81, 101) oder der zumindest eine Kunststoff des Hohlkörpers (1, 21, 41, 61, 81, 101) mindestens einen antiseptischen Wirkstoff enthält oder mit mindestens einem antiseptischen Wirkstoff beschichtet ist.

12. Medizinischer Platzhalter nach einem der vorangehenden Ansprüche, wobei in zumindest einer Wandung des Hohlkörpers (1, 21, 41, 61, 81, 101) oder des zumindest einen Kunststoffs des Hohlkörpers (1, 21, 41, 61, 81, 101) Durchführungen angeordnet sind, die in Richtung der Außenseite des Hohlkörpers (1, 21, 41, 61, 81, 101) weisende Öffnungen aufweisen und die zumindest einen Kanal mit der Umgebung des Hohlkörpers (1, 21, 41, 61, 81, 101) für Flüssigkeiten und Gase durchlässig verbinden, wobei der zumindest eine Kanal zum Einleiten von Flüssigkeiten in den Hohlkörper (1, 21, 41, 61, 81, 101) zu den Durchführungen vorgesehen ist, wobei vorzugsweise der zumindest eine Kanal mit einem Zuleitungsschlauch für pharmazeutische Wirkstofflösungen verbunden ist, wobei besonders bevorzugt an dem Zuleitungsschlauch ein Anschlussadapter, insbesondere ein Luer-Lock-Adapter angeordnet ist.

13. Medizinischer Platzhalter nach einem der vorangehenden Ansprüche, wobei der Hohlkörper (1, 21, 41, 61, 81, 101) im nicht aufgeblasenen Zustand an dem Gaseinleitungsschlauch (2, 22, 42, 62, 82, 102) und/oder an dem Gasausleitungsschlauch (3, 23, 43, 63, 83, 103) anliegt, wobei vorzugsweise der Hohlkörper (1, 21, 41, 61, 81, 101) oder der zumindest eine Kunststoff nach Art eines Ballons ausblasbar ist.

14. Verfahren zum Begasen einer Oberfläche eines medizinischen Platzhalters, insbesondere eines medizinischen Platzhalters nach einem der vorangehenden Ansprüche, wobei das Verfahren nicht zur medizinischen Behandlung eines menschlichen oder tierischen Körpers durchgeführt wird, aufweisend die folgenden Schritte:
A) Expandieren eines Hohlkörpers (1, 21, 41, 61, 81, 101) durch Einleiten einer Spülflüssigkeit oder eines Spülgases enthaltend Sauerstoff in einen Innenraum des Hohlkörpers (1, 21, 41, 61, 81, 101), so dass der Innenraum des Hohlkörpers (1, 21, 41, 61, 81, 101) vergrößert wird;
B) Abgeben von gasförmigem Sauerstoff aus der Spülflüssigkeit oder dem Spülgas durch einen den Innenraum begrenzenden Kunststoff hindurch an die Umgebung des Hohlkörpers (1, 21, 41, 61, 81, 101);
C) Aufnehmen von gasförmigem Kohlendioxid aus der Umgebung des Hohlkörpers (1, 21, 41, 61, 81, 101) durch den den Innenraum begrenzenden Kunststoff hindurch in die Spülflüssigkeit oder das Spülgas;
D) Hindurchleiten der Spülflüssigkeit oder des Spülgases durch den Innenraum des Hohlkörpers (1, 21, 41, 61, 81, 101) und Ableiten der Spülflüssigkeit oder des Spülgases aus dem Innenraum.

15. Verfahren nach Anspruch 14, wobei vor Schritt A) der Hohlkörper (1, 21, 41, 61, 81, 101) in einen Hohlraum eingeführt wird, wobei während Schritt A) die Form des aufgeblasenen Hohlkörpers (1, 21, 41, 61, 81, 101) sich zumindest bereichsweise an die Form des Hohlraums anpasst und während der Schritte B) und C) zumindest bereichsweise an dem Hohlraum anliegt, und/oder in Schritt A) der Hohlkörper (1, 21, 41, 61, 81, 101) aufgeblasen wird, bis ein Mindestdruck erreicht wird, wobei sich bei Erreichen des Mindestdrucks ein Überdruckventil (5, 25, 45) öffnet, durch das die Spülflüssigkeit oder das Spülgas aus dem Innenraum des Hohlkörpers (1, 21, 41, 61, 81, 101) während Schritt D) abgeleitet wird.

## Claims

1. A medical placeholder for temporary implantation into bone cavities comprising at least one hollow body (1, 21, 41, 61, 81, 101), which is expandable and defines an interior space in the interior of the hollow body (1, 21, 41, 61, 81, 101),
a gas infeed hose (2, 22, 42, 62, 82, 102), which is connected or connectable in gas-permeable manner with the interior space of the hollow body (1, 21, 41, 61, 81, 101), a gas discharge hose (3, 23, 43, 63, 83, 103), which is connected or connectable in gas-permeable manner with the interior space of the hollow body (1, 21, 41, 61, 81, 101),
wherein the hollow body (1, 21, 41, 61, 81, 101) consists at least in areas or completely of at least one plastics material, wherein the hollow body (1, 21, 41, 61, 81, 101) or the at least one plastics material of the hollow body (1, 21, 41, 61, 81, 101) has a permeability coefficient for oxygen of greater than or equal to 0.5 cm³/(m²*d*bar) and a permeability coefficient for carbon dioxide of greater than or equal to 0.5 cm³/(m²*d*bar), and
wherein a one-way valve is arranged in the gas infeed hose (2, 22, 42, 62, 82, 102) and a pressure relief valve is arranged in the gas discharge hose (3, 23, 43, 63, 83, 103).

2. The medical placeholder according to Claim 1, wherein
the hollow body (1, 21, 41, 61, 81, 101) or the at least one plastics material of the hollow body (1, 21, 41, 61, 81, 101) has a permeability coefficient for oxygen of greater than or equal to 1 cm³/(m²*d*bar) and a permeability coefficient for carbon dioxide of greater than or equal to 1 cm³/(m²*d*bar).

3. The medical placeholder according to Claim 1 or 2, wherein
an inflow opening (10, 30, 50, 70, 90, 110) of the gas infeed hose (2, 22, 42, 62, 82, 102), with which the gas infeed hose (2, 22, 42, 62, 82, 102) opens into the interior space, is arranged spatially separately from an outflow opening (11, 31, 51, 71, 91, 111) of the gas discharge hose (3, 23, 43, 63, 83, 103), wherein the outflow opening (11, 31, 51, 71, 91, 111) forms the point where the interior space opens into the gas discharge hose (3, 23, 43, 63, 83, 103).

4. The medical placeholder according to Claim 3, wherein
the inflow opening (10, 30, 50, 70, 90, 110) of the gas infeed hose (2, 22, 42, 62, 82, 102) is arranged at a first end of the hollow body (1, 21, 41, 61, 81, 101) and the outflow opening (11, 31, 51, 71, 91, 111) is arranged at a second end of the hollow body (1, 21, 41, 61, 81, 101) opposite the first end.

5. The medical placeholder according to any one of the preceding claims, wherein the gas infeed hose (22) and the gas discharge hose (23) are arranged in places coaxially to one another, wherein the two are preferably guided jointly out of the hollow body (21) through a feedthrough.

6. The medical placeholder according to any one of the preceding claims, wherein the gas infeed hose (2, 22, 42, 62, 82, 102) and the gas discharge hose (3, 23, 43, 63, 83, 103) are connected to one another.

7. The medical placeholder according to any one of the preceding claims, wherein the gas infeed hose (22, 42) and the gas discharge hose (23, 43) have a common outer hose (38, 58), wherein the common outer hose (38, 58) is preferably subdivided by a partition which defines the gas infeed hose (22, 42) on one side and the gas discharge hose (23, 43) on the other side.

8. The medical placeholder according to any one of the preceding claims, wherein a sterile filter which is impermeable to microbes but permeable to gases is arranged in the gas infeed hose (2, 22, 42, 62, 82, 102) and/or the gas discharge hose (3, 23, 43, 63, 83, 103).

9. The medical placeholder according to Claim 8, wherein
the sterile filter is arranged in the gas infeed hose (2, 22, 42, 62, 82, 102) or the gas discharge hose (3, 23, 43, 63, 83, 103) downstream of the one-way valve in the direction of flow or the sterile filters are arranged in the gas infeed hose (2, 22, 42, 62, 82, 102) and in the gas discharge hose (3, 23, 43, 63, 83, 103) downstream of the one-way valve and downstream of the pressure relief valve.

10. The medical placeholder according to any one of the preceding claims, wherein the opening pressure of the pressure relief valve is adjustable, and/or
the gas infeed hose (2, 22, 42, 62, 82, 102) and the gas discharge hose (3, 23, 43, 63, 83, 103) consist of a material which is not permeable to oxygen and carbon dioxide, preferably of a plastics material which is not permeable to oxygen and carbon dioxide.

11. The medical placeholder according to any one of the preceding claims, wherein the hollow body (1, 21, 41, 61, 81, 101) or the at least one plastics material of the hollow body (1, 21, 41, 61, 81, 101) contains at least one antiseptic active ingredient or is coated with at least one antiseptic active ingredient.

12. The medical placeholder according to any one of the preceding claims, wherein feedthroughs are arranged in at least one wall of the hollow body (1, 21, 41, 61, 81, 101) or of the at least one plastics material of the hollow body (1, 21, 41, 61, 81, 101), said feedthroughs having openings pointing in the direction of the outside of the hollow body (1, 21, 41, 61, 81, 101) and connecting at least one duct with the surroundings of the hollow body (1, 21, 41, 61, 81, 101) in a manner permeable to liquids and gases, wherein the at least one duct is provided for feeding liquids into the hollow body (1, 21, 41, 61, 81, 101) to the feedthroughs, wherein the at least one duct is preferably connected with a feed hose for pharmaceutical active ingredient solutions, wherein a connection adapter, in particular a Luer-Lock adapter, is particularly preferably arranged at the feed hose.

13. The medical placeholder according to any one of the preceding claims, wherein hollow body (1, 21, 41, 61, 81, 101) rests in the uninflated state against the gas infeed hose (2, 22, 42, 62, 82, 102) and/or against the gas discharge hose (3, 23, 43, 63, 83, 103), wherein the hollow body (1, 21, 41, 61, 81, 101) or the at least one plastics material is preferably inflatable in the manner of a balloon.

14. A method for gas-flushing a surface of a medical placeholder, in particular of a medical placeholder according to any one of the preceding claims, wherein the method is not performed for medical treatment of a human or animal body, having the following steps:
A) Expanding a hollow body (1, 21, 41, 61, 81, 101) by feeding a flushing liquid containing oxygen or a flushing gas containing oxygen into an interior space of the hollow body (1, 21, 41, 61, 81, 101), thereby enlarging the interior space of the hollow body (1, 21, 41, 61, 81, 101);
B) Delivering gaseous oxygen from the flushing liquid or the flushing gas through a plastics material defining the interior space to the surroundings of the hollow body (1, 21, 41, 61, 81, 101);
C) Absorbing gaseous carbon dioxide from the surroundings of the hollow body (1, 21, 41, 61, 81, 101) through the plastics material defining the interior space into the flushing liquid or the flushing gas;
D) Passing the flushing liquid or the flushing gas through the interior space of the hollow body (1, 21, 41, 61, 81, 101) and discharging the flushing liquid or flushing gas out of the interior space.

15. The method according to Claim 14, wherein
the hollow body (1, 21, 41, 61, 81, 101) is introduced into a cavity prior to step A), wherein during step A) the shape of the inflated hollow body (1, 21, 41, 61, 81, 101) adapts at least in places to the shape of the cavity and during steps B) and C) rests at least in places against the cavity, and/or
the hollow body (1, 21, 41, 61, 81, 101) is inflated in step A) until a minimum pressure is reached, wherein a pressure relief valve (5, 25, 45) opens once the minimum pressure is reached, the flushing liquid or the flushing gas being discharged through this pressure relief valve from the interior space of the hollow body (1, 21, 41, 61, 81, 101) during step D).

## Revendications

1. Écarteur médical destiné à être implanté temporairement dans des cavités osseuses, présentant au moins un corps creux (1, 21, 41, 61, 81, 101) qui peut être dilaté et qui délimite un espace interne à l'intérieur du corps creux (1, 21, 41, 61, 81, 101), un tuyau d'introduction de gaz (2, 22, 42, 62, 82, 102) qui est relié ou peut être relié de manière perméable aux gaz à l'espace interne du corps creux (1, 21, 41, 61, 81, 101), un tuyau d'évacuation de gaz (3, 23, 43, 63, 83, 103) qui est relié ou peut être relié de manière perméable aux gaz à l'espace interne du corps creux (1, 21, 41, 61, 81, 101), le corps creux (1, 21, 41, 61, 81, 101) étant constitué, au moins dans certaines régions ou complètement, d'au moins un matériau synthétique, le corps creux (1, 21, 41, 61, 81, 101) ou l'au moins un matériau synthétique du corps creux (1, 21, 41, 61, 81, 101) présentant un coefficient de perméabilité à l'oxygène supérieur ou égal à 0,5 cm³/(m²*d*bar) et un coefficient de perméabilité au dioxyde de carbone supérieur ou égal à 0,5 cm³/(m²*d*bar), et
une soupape unidirectionnelle étant disposée dans le tuyau d'introduction de gaz (2, 22, 42, 62, 82, 102) et une soupape de surpression étant disposée dans le tuyau d'évacuation de gaz (3, 23, 43, 63, 83, 103).

2. Écarteur médical selon la revendication 1,
le corps creux (1, 21, 41, 61, 81, 101) ou l'au moins un matériau synthétique du corps creux (1, 21, 41, 61, 81, 101) présentant un coefficient de perméabilité à l'oxygène supérieur ou égal à 1 cm³/(m²*d*bar) et un coefficient de perméabilité au dioxyde de carbone supérieur ou égal à 1 cm³/(m²*d*bar).

3. Écarteur médical selon la revendication 1 ou 2,
une ouverture d'admission (10, 30, 50, 70, 90, 110) du tuyau d'introduction de gaz (2, 22, 42, 62, 82, 102), avec laquelle le tuyau d'introduction de gaz (2, 22, 42, 62, 82, 102) débouche dans l'espace interne, étant disposée de manière à être spatialement séparée d'une ouverture de sortie (11, 31, 51, 71, 91, 111) du tuyau d'évacuation de gaz (3, 23, 43, 63, 83, 103), l'ouverture de sortie (11, 31, 51, 71, 91, 111) formant l'embouchure de l'espace interne dans le tuyau d'évacuation de gaz (3, 23, 43, 63, 83, 103).

4. Écarteur médical selon la revendication 3,
l'ouverture d'admission (10, 30, 50, 70, 90, 110) du tuyau d'introduction de gaz (2, 22, 42, 62, 82, 102) étant disposée au niveau d'une première extrémité du corps creux (1, 21, 41, 61, 81, 101) et l'ouverture de sortie (11, 31, 51, 71, 91, 111) étant disposée au niveau d'une seconde extrémité du corps creux (1, 21, 41, 61, 81, 101) opposée à la première extrémité.

5. Écarteur médical selon l'une des revendications précédentes,
le tuyau d'introduction de gaz (22) et le tuyau d'évacuation de gaz (23) étant disposés dans certaines régions de manière coaxiale l'un par rapport à l'autre, de préférence, les deux tuyaux étant guidés conjointement à travers un passage hors du cors creux (21).

6. Écarteur médical selon l'une des revendications précédentes,
le tuyau d'introduction de gaz (2, 22, 42, 62, 82, 102) et le tuyau d'évacuation de gaz (3, 23, 43, 63, 83, 103) étant reliés l'un à l'autre.

7. Écarteur médical selon l'une des revendications précédentes,
le tuyau d'introduction de gaz (22, 42) et le tuyau d'évacuation de gaz (23, 43) présentant un tuyau externe commun (38, 58), de manière particulièrement préférée, le tuyau externe commun (38, 58) étant divisé par une paroi de séparation qui délimite le tuyau d'introduction de gaz (22, 42) sur un côté et délimite le tuyau d'évacuation de gaz (23, 43) sur l'autre côté.

8. Écarteur médical selon l'une des revendications précédentes,
un filtre stérile imperméable aux germes mais perméable aux gaz étant disposé dans le tuyau d'introduction de gaz (2, 22, 42, 62, 82, 102) et/ou dans le tuyau d'évacuation de gaz (3, 23, 43, 63, 83, 103).

9. Écarteur médical selon la revendication 8,
le filtre stérile étant disposé dans le tuyau d'introduction de gaz (2, 22, 42, 62, 82, 102) ou dans le tuyau d'évacuation de gaz (3, 23, 43, 63, 83, 103) en aval, dans le sens d'écoulement, de la soupape unidirectionnelle ou les filtres stériles étant disposés dans le tuyau d'introduction de gaz (2, 22, 42, 62, 82, 102) et dans le tuyau d'évacuation de gaz (3, 23, 43, 63, 83, 103) en aval de la soupape unidirectionnelle et en aval de la soupape de surpression.

10. Écarteur médical selon l'une des revendications précédentes,
la pression d'ouverture de la soupape de surpression étant réglable, et/ou le tuyau d'introduction de gaz (2, 22, 42, 62, 82, 102) et le tuyau d'évacuation de gaz (3, 23, 43, 63, 83, 103) étant constitués d'un matériau non perméable à l'oxygène et au dioxyde de carbone, de préférence d'un matériau synthétique non perméable à l'oxygène et au dioxyde de carbone.

11. Écarteur médical selon l'une des revendications précédentes,
le corps creux (1, 21, 41, 61, 81, 101) ou l'au moins un matériau synthétique du corps creux (1, 21, 41, 61, 81, 101) contenant au moins un principe actif antiseptique ou étant revêtu d'au moins un principe actif antiseptique.

12. Écarteur médical selon l'une des revendications précédentes,
des passages étant disposés dans au moins une paroi du corps creux (1, 21, 41, 61, 81, 101) ou de l'au moins un matériau synthétique du corps creux (1, 21, 41, 61, 81, 101), lesquels passages présentent des ouvertures orientées en direction du côté externe du corps creux (1, 21, 41, 61, 81, 101) et relient au moins un canal à l'environnement du corps creux (1, 21, 41, 61, 81, 101) de manière perméable aux liquides et aux gaz, l'au moins un canal étant prévu pour l'introduction de liquides dans le corps creux (1, 21, 41, 61, 81, 101) vers les passages, de préférence, l'au moins un canal étant relié à un tuyau d'amenée pour des solutions de principe actif pharmaceutiques, de manière particulièrement préférée, un adaptateur de raccordement, en particulier un adaptateur Luer-Lock, étant disposé au niveau du tuyau d'amenée.

13. Écarteur médical selon l'une des revendications précédentes,
le corps creux (1, 21, 41, 61, 81, 101) reposant, dans l'état non gonflé, contre le tuyau d'introduction de gaz (2, 22, 42, 62, 82, 102) et/ou contre le tuyau d'évacuation de gaz (3, 23, 43, 63, 83, 103), de préférence, le corps creux (1, 21, 41, 61, 81, 101) ou l'au moins un matériau synthétique pouvant être gonflé à la manière d'un ballon.

14. Procédé permettant l'aération d'une surface d'un écarteur médical, en particulier d'un écarteur médical selon l'une des revendications précédentes, le procédé n'étant pas réalisé pour le traitement médical d'un corps humain ou animal, présentant les étapes suivantes :
A) dilatation d'un corps creux (1, 21, 41, 61, 81, 101) par introduction d'un liquide de rinçage ou d'un gaz de rinçage contenant de l'oxygène dans un espace interne du corps creux (1, 21, 41, 61, 81, 101), de sorte que l'espace interne du corps creux (1, 21, 41, 61, 81, 101) est agrandi ;
B) distribution d'oxygène gazeux en provenance du liquide de rinçage ou du gaz de rinçage à l'environnement du corps creux (1, 21, 41, 61, 81, 101) à travers un matériau synthétique délimitant l'espace interne ;
C) absorption de dioxyde de carbone gazeux en provenance de l'environnement du corps creux (1, 21, 41, 61, 81, 101) dans le liquide de rinçage ou le gaz de rinçage à travers le matériau synthétique délimitant l'espace interne ;
D) guidage du liquide de rinçage ou du gaz de rinçage à travers l'espace interne du corps creux (1, 21, 41, 61, 81, 101) et évacuation du liquide de rinçage ou du gaz de rinçage hors de l'espace interne.

15. Procédé selon la revendication 14,
avant l'étape A), le corps creux (1, 21, 41, 61, 81, 101) étant introduit dans un espace creux, pendant l'étape A), la forme du corps creux (1, 21, 41, 61, 81, 101) gonflé s'adaptant au moins dans certaines régions à la forme de l'espace creux et, pendant les étapes B) et C), reposant au moins dans certaines régions contre l'espace creux, et/ou dans l'étape A), le corps creux (1, 21, 41, 61, 81, 101) étant gonflé jusqu'à atteindre une pression minimale, une soupape de surpression (5, 25, 45) s'ouvrant lorsque la pression minimale est atteinte, à travers laquelle soupape de surpression le liquide de rinçage ou le gaz de rinçage est évacué hors de l'espace interne du corps creux (1, 21, 41, 61, 81, 101) pendant l'étape D).
